(19) **Europäisches Patentamt**
**European Patent Office**
**Office européen des brevets**

(11) Veröffentlichungsnummer: **0 400 545 B1**

(12) **EUROPÄISCHE PATENTSCHRIFT**

(45) Veröffentlichungstag der Patentschrift: **15.03.95**

(51) Int. Cl.6: **C12N 15/58**, C07H 21/04, C12P 21/00, C07K 14/00, A61K 38/46

(21) Anmeldenummer: **90110096.6**

(22) Anmeldetag: **28.05.90**

Die Akte enthält technische Angaben, die nach dem Eingang der Anmeldung eingereicht wurden und die nicht in dieser Patentschrift enthalten sind.

(54) **Gewebs-Plasminogen-Aktivator-Derivat.**

(30) Priorität: **31.05.89 DE 3917781**
**14.07.89 DE 3923339**

(43) Veröffentlichungstag der Anmeldung:
**05.12.90 Patentblatt 90/49**

(45) Bekanntmachung des Hinweises auf die Patenterteilung:
**15.03.95 Patentblatt 95/11**

(84) Benannte Vertragsstaaten:
**AT BE CH DE DK ES FR GB GR IT LI LU NL SE**

(56) Entgegenhaltungen:
**EP-A- 0 236 209**
**EP-A- 0 242 836**
**EP-A- 0 277 313**
**EP-A- 0 302 456**

(73) Patentinhaber: **BOEHRINGER MANNHEIM GMBH**

**D-68298 Mannheim (DE)**

(72) Erfinder: **Stern, Anne, Dr. rer. nat.**
**Karwendelstrasse 10**
**D-8122 Penzberg (DE)**

Erfinder: **Kohnert, Ulrich, Dr. rer. nat.**
**Heubachweg 6**
**D-8121 Habach (DE)**
Erfinder: **Rudolph, Rainer, Dr. rer. nat.**
**Färbergasse 19**
**D-8120 Weilheim (DE)**
Erfinder: **Fischer, Stephan, Dr. rer. nat.**
**Jakobifeldweg 11**
**D-8121 Polling (DE)**
Erfinder: **Martin, Ulrich, Dr. med.**
**D 3, 4**
**D-6800 Mannheim 1 (DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**D-81635 München (DE)**

**Beschreibung**

Die Erfindung betrifft ein neues t-PA-Derivat, eine DNA-Sequenz, welche für das neue t-PA-Derivat kodiert, Expressionsplasmide, welche eine für das t-PA-Derivat kodierende DNA-Sequenz aufweisen sowie ein Verfahren zur Herstellung derartiger Plasmide, Verfahren zur Herstellung des t-PA-Derivats und ein Mittel zur Gerinnselauflösung, enthaltend das t-PA-Derivat.

Geronnenes Blut enthält als Hauptkomponente der Proteinmatrix polymeres Fibrin. Fibrin wird durch ein fibrinolytisches System unter physiologischen Bedingungen in einer Reaktionskaskade aufgelöst, die der der Blutgerinnung ähnelt. Die zentrale Reaktion ist hierbei die Aktivierung von Plasminogen zu Plasmin, die z.B. durch den Gewebsplasminogenaktivator t-PA (tissue type plasminogen activator) bewirkt wird. Plasmin wiederum löst Fibrin, das die Hauptkomponente der Proteinmatrix von geronnenem Blut ist. Die enzymatische Aktivität von natürlichem oder aus Eukaryonten gentechnologisch gewonnenem t-PA, nämlich die katalytische Aktivierung von Plasminogen zu Plasmin, ist in Abwesenheit von Fibrin oder Fibrinogen-Spaltprodukten sehr gering, kann jedoch bei Anwesenheit dieser Proteine deutlich gesteigert werden, nämlich um mehr als den Faktor 10.

t-PA wird im Blut durch vorhandene Proteasen in eine A-und eine B-Kette gespalten. Die beiden Teilketten bleiben über eine Cysteinbrücke verbunden. Die Stimulierbarkeit der Aktivität des t-PA ist ein entscheidender Vorteil gegenüber anderen bekannten Plasminogen-Aktivatoren, wie z.B. Urokinase oder Streptokinase (vgl. z.B. M. Hoylaerts et al., J. Biol. Chem. 257 (1982), 2912-2919; W. Nieuwenhuizen et al., Biochem. Biophys. Acta, 755 (1983), 531-533).

Der Wirkungsmechanismus von t-PA in vivo ist beispielsweise in Korniger und Collen, Thromb. Hämostasis 46 (1981), 561-565, beschrieben. Die auf die Fibrinoberfläche focussierte Aktivität des Enzyms läßt es als geeignetes Mittel zur Bekämpfung von pathologischen Aderverschlüssen (z.B. beim Herzinfarkt) erscheinen, was durch klinische Versuche größtenteils bestätigt werden konnte (Collen et. al., Circulation 70 (1984), 1012; Circulation 73 (1986), 511).

Als Nachteil von t-PA muß jedoch die rasche Abnahme seiner Plasmakonzentration (Clearance-Rate) angesehen werden. Die Folge daraus ist, daß eine relativ große Menge an t-PA erforderlich ist, um in vivo eine effektive Lysierung von Thromben zu erzielen. Die hohen Behandlungsdosen wiederum haben Nebenwirkungen, wie z.B. Blutungen, zur Folge.

In der EP 0 196 920 ist ein natürliches Abbauprodukt von t-PA beschrieben, welches nur noch die Kringel 2-und die Protease-Domänen des t-PA enthält und dessen N-terminale Aminosäure Alanin 160 der von Pennica et al., Nature 301 (1983), 214-221 beschriebenen Aminosäuresequenz ist. Die Clearance-Rate dieses t-PA-Abbauprodukts ist jedoch nicht wesentlich von der des natürlichen t-PA verschieden. Erst durch eine chemische Modifikation des katalytischen Bereichs durch Anbringen einer blockierenden Gruppe kann hier eine Verbesserung erreicht werden.

Aufgabe der Erfindung ist es daher, t-PA derart zu verändern, daß das entstandene Derivat eine stark reduzierte Clearance-Rate und damit eine verlängerte Halbwertszeit im Blutplasma aufweist. Es sollen hierbei die Thromben-lysierende Wirkung sowie die Stimulierbarkeit durch Fibrin erhalten bleiben.

Gegenstand der Erfindung ist daher ein Gewebsplasminogen-Aktivator (t-PA-Derivat, in den Beispielen auch K1K2P genannt), der dadurch gekennzeichnet ist, daß er nicht glykosyliert ist und aus der folgenden Aminosäuresequenz besteht:

(M)

```
Ser Tyr Gln Val Ile Asp Thr Arg Ala Thr Cys Tyr Glu Asp Gln Gly
                  5                  10                    15

Ile Ser Tyr Arg Gly Thr Trp Ser Thr Ala Glu Ser Gly Ala Glu Cys
            20              25                    30

Thr Asn Trp Asn Ser Ser Ala Leu Ala Gln Lys Pro Tyr Ser Gly Arg
        35                  40              45

Arg Pro Asp Ala Ile Arg Leu Gly Leu Gly Asn His Asn Tyr Cys Arg
    50                  55                  60

Asn Pro Asp Arg Asp Ser Lys Pro Trp Cys Tyr Val Phe Lys Ala Gly
 65                  70                  75                    80

Lys Tyr Ser Ser Glu Phe Cys Ser Thr Pro Ala Cys Ser Glu Gly Asn
              85                  90                    95

Ser Asp Cys Tyr Phe Gly Asn Gly Ser Ala Tyr Arg Gly Thr His Ser
            100             105                 110

Leu Thr Glu Ser Gly Ala Ser Cys Leu Pro Trp Asn Ser Met Ile Leu
        115             120                 125

Ile Gly Lys Val Tyr Thr Ala Gln Asn Pro Ser Ala Gln Ala Leu Gly
    130             135                 140

Leu Gly Lys His Asn Tyr Cys Arg Asn Pro Asp Gly Asp Ala Lys Pro
145             150                 155                     160

Trp Cys His Val Leu Lys Asn Arg Arg Leu Thr Trp Glu Tyr Cys Asp
            165                 170                 175

Val Pro Ser Cys Ser Thr Cys Gly Leu Arg Gln Tyr Ser Gln Pro Gln
            180             185                 190
```

3

```
Phe Arg Ile Lys Gly Gly Leu Phe Ala Asp Ile Ala Ser His Pro Trp
        195             200             205

Gln Ala Ala Ile Phe Ala Lys His Arg Arg Ser Pro Gly Glu Arg Phe
        210             215             220

Leu Cys Gly Gly Ile Leu Ile Ser Ser Cys Trp Ile Leu Ser Ala Ala
225                     230             235                 240

His Cys Phe Gln Glu Arg Phe Pro Pro His His Leu Thr Val Ile Leu
            245             250                 255

Gly Arg Thr Tyr Arg Val Val Pro Gly Glu Glu Glu Gln Lys Phe Glu
            260             265                 270

Val Glu Lys Tyr Ile Val His Lys Glu Phe Asp Asp Asp Thr Tyr Asp
        275                 280                 285

Asn Asp Ile Ala Leu Leu Gln Leu Lys Ser Asp Ser Ser Arg Cys Ala
        290                 295             300

Gln Glu Ser Ser Val Val Arg Thr Val Cys Leu Pro Pro Ala Asp Leu
305                 310                 315                 320

Gln Leu Pro Asp Trp Thr Glu Cys Glu Leu Ser Gly Tyr Gly Lys His
            325                 330                 335

Glu Ala Leu Ser Pro Phe Tyr Ser Glu Arg Leu Lys Glu Ala His Val
            340                 345             350

Arg Leu Tyr Pro Ser Ser Arg Cys Thr Ser Gln His Leu Leu Asn Arg
            355             360             365

Thr Val Thr Asp Asn Met Leu Cys Ala Gly Asp Thr Arg Ser Gly Gly
        370             375             380

Pro Gln Ala Asn Leu His Asp Ala Cys Gln Gly Asp Ser Gly Gly Pro
385             390             395             400

Leu Val Cys Leu Asn Asp Gly Arg Met Thr Leu Val Gly Ile Ile Ser
            405             410             415

Trp Gly Leu Gly Cys Gly Gln Lys Asp Val Pro Gly Val Tyr Thr Lys
            420             425             430

Val Thr Asn Tyr Leu Asp Trp Ile Arg Asp Asn Met Arg Pro
        435             440             445
```

Überraschenderweise wurde festgestellt, daß die Deletion der übrigen, im nativen t-PA vorhandenen Bereiche keinen Einfluß auf die thrombolytische Wirksamkeit des Proteins hat und die fibrinabhängige Stimulierbarkeit des Muteins gleich der von nativem t-PA ist.

Ein weiterer Gegenstand der Erfindung ist eine DNA-Sequenz, die für das erfindungsgemäße t-PA-Derivat kodiert und die folgende Sequenz enthält:

```
ATGTCTTACCAAGTGATCGATACCAGGGCCACGTGCTACGAGGACCAGGGCATCAGCTAC
AGGGGCACGTGGAGCACAGCGGAGAGTGGCGCCGAGTGCACCAACTGGAACAGCAGCGCG
TTGGCCCAGAAGCCCTACAGCGGGCGGAGGCCAGACGCCATCAGGCTGGGCCTGGGGAAC
CACAACTACTGCAGAAACCCAGATCGAGACTCAAAGCCCTGGTGCTACGTCTTTAAGGCG
GGGAAGTACAGCTCAGAGTTCTGCAGCACCCCTGCCTGCTCTGAGGGAAACAGTGACTGC
TACTTTGGGAATGGGTCAGCCTACCGTGGCACGCACAGCCTCACCGAGTCGGGTGCCTCC
TGCCTCCCGTGGAATTCCATGATCCTGATAGGCAAGGTTTACACAGCACAGAACCCCAGT
GCCCAGGCACTGGGCCTGGGCAAACATAATTACTGCCGGAATCCTGATGGGGATGCCAAG
CCCTGGTGCCACGTGCTGAAGAACCGCAGGCTGACGTGGGAGTACTGTGATGTGCCCTCC
TGCTCCACCTGCGGCCTGAGACAGTACAGCCAGCCTCAGTTTCGCATCAAAGGAGGGCTC
TTCGCCGACATCGCCTCCCACCCCTGGCAGGCTGCCATCTTTGCCAAGCACAGGAGGTCG
CCCGGAGAGCGGTTCCTGTGCGGGGGCATACTCATCAGCTCCTGCTGGATTCTCTCTGCC
GCCCACTGCTTCCAGGAGAGGTTTCCGCCCCACCACCTGACGGTGATCTTGGGCAGAACA
TACCGGGTGGTCCCTGGCGAGGAGGAGCAGAAATTTGAAGTCGAAAAATACATTGTCCAT
AAGGAATTCGATGATGACACTTACGACAATGACATTGCGCTGCTGCAGCTGAAATCGGAT
TCGTCCCGCTGTGCCCAGGAGAGCAGCGTGGTCCGCACTGTGTGCCTTCCCCCGGCGGAC
CTGCAGCTGCCGGACTGGACGGAGTGTGAGCTCTCCGGCTACGGCAAGCATGAGGCCTTG
TCTCCTTTCTATTCGGAGCGGCTGAAGGAGGCTCATGTCAGACTGTACCCATCCAGCCGC
TGCACATCACAACATTTACTTAACAGAACAGTCACCGACAACATGCTGTGTGCTGGAGAC
ACTCGGAGCGGCGGGCCCCAGGCAAACTTGCACGACGCCTGCCAGGGCGATTCGGGAGGC
CCCCTGGTGTGTCTGAACGATGGCCGCATGACTTTGGTGGGCATCATCAGCTGGGGCCTG
GGCTGTGGACAGAAGGATGTCCCGGGTGTGTACACAAAGGTTACCAACTACCTAGACTGG
ATTCGTGACAACATGCGACCG   1341
```

Die erfindungsgemäße DNA-Sequenz dient zur Expression des erfindungsgemäßen t-PA-Derivats, wenn sie auf einem Expressionsplasmid vorhanden ist. Ein derartiges Expressionsplasmid ist ein weiterer Gegenstand der Erfindung, ebenso wie ein Expressionsplasmid, das eine hiervon abweichende DNA-Sequenz aufweist, die jedoch ebenfalls für das erfindungsgemäße t-PA-Derivat kodiert. Aufgrund der Degeneration des genetischen Codes sind hierfür auch von der gezeigten DNA-Sequenz abweichende Sequenzen geeignet.

Das Expressionsplasmid enthält außer einem Replikationsursprung (origin of replication) vorzugsweise neben der für das t-PA-Derivat kodierenden Sequenz zusätzlich noch eine regulierbare Promotorstruktur (z.B. tac-Promotor), einen effizienten Terminator (z.B. fd), einen Selektionsmarker (z.B. ß-Lactamase-Gen).

Ein weiterer Gegenstand der Erfindung ist das Plasmid pA33. Die Herstellung dieses Plasmids ist im Beispiel 1 beschrieben, es enthält eine DNA-Sequenz, die für das erfindungsgemäße t-PA-Derivat kodiert.

Wiederum ein weiterer Gegenstand der Erfindung ist ein Verfahren zur Herstellung eines der erfindungsgemäßen Expressionsplasmide, das dadurch gekennzeichnet ist, daß man eine DNA-Sequenz, die für das erfindungsgemäße t-PA-Protein oder ein Derivat davon, welches über die Kringel I, Kringel II- und die Protease-Domänen hinaus noch weitere Bereiche des t-PA-Proteins aufweist, kodiert, in ein Plasmid einbringt und über gerichtete Mutagenese diejenigen Bereiche entfernt, die für Aminosäuren kodieren, die im erfindungsgemäßen t-PA-Derivat nicht vorhanden sind.

Die Auswahl des Plasmids, in das die für das erfindungsgemäße t-PA-Derivat kodierende DNA-Sequenz eingebracht wird, ist abhängig von den später zur Expression des Derivats verwendeten Wirtszellen. Geeignete Plasmide sowie die Minimalanforderungen, die an ein derartiges Plasmid gestellt werden (z.B. Replikationsursprung, Restriktionsschnittstelle) sind dem Fachmann bekannt. Im Rahmen der Erfindung können jedoch anstelle eines Plasmids auch ein Cosmid, die replikative, doppelsträngige Form von Phagen (λ, M13), und andere dem Fachmann bekannte Vektoren verwendet werden. Die Methode der gerichteten Mutagenese (site-directed mutagenesis) ist von Morinaga et al., Biotechnology 21, (1984), 634, beschrieben, und wird im wesentlichen wie dort beschrieben ausgeführt.

Wiederum ein weiterer Gegenstand der Erfindung ist ein Verfahren zur Herstellung eines erfindungsgemäßen t-PA-Derivats, das dadurch gekennzeichnet ist, daß man eines der erfindungsgemäßen Plasmide in geeigneten Wirtszellen exprimiert und das Produkt aus dem Kulturmedium oder nach Aufschluß der Wirtszellen aus der Flüssigkeit, in der der Aufschluß durchgeführt wurde (z.B. Puffer oder aber auch das Kulturmedium) gewinnt. Vorzugsweise werden zur Herstellung des erfindungsgemäßen t-PA-Derivats als Wirtszellen Prokaryontenzellen verwendet. Hierbei ist es wiederum besonders bevorzugt, daß man die sich hierbei bildenden sogenannten "inclusion bodies" (unlösliche Proteinaggregate) zunächst von den löslichen Zellpartikeln abtrennt, die t-PA enthaltenden inclusion bodies durch Behandlung mit Guanidin-Hydrochlorid solubilisiert, sie anschließend mit oxidiertem Glutathion derivatisiert und zuletzt das t-PA-Derivat durch Zugabe von L-Arginin und Guanidinhydrochlorid renaturiert. Genaue Vorschriften zur Aktivierung von t-PA aus "inclusion bodies" sind beispielsweise in den Patentanmeldungen EP-A 0 219 874 und EP-A 0 241 022 offenbart. Jegliche andere Verfahren zur Gewinnung des aktiven Proteins aus "inclusion bodies" können jedoch erfindungsgemäß ebenso eingesetzt werden.

Das erfindungsgemäße Verfahren wird bevorzugt in Anwesenheit von L-Arginin, insbesondere in einer Konzentration von 25 bis 1000 mmol/l, durchgeführt.

Die erfindungsgemäße Abtrennung von Fremdprotein über eine Affinitätschromatographie wird in einer bevorzugten Ausführungsform der Erfindung über eine ETI (Erythrina-Trypsin-Inhibitor)-Adsorbersäule durchgeführt. Hierbei ist ETI auf einem Trägermaterial (Adsorber), wie z.B. BrCN-Sepharose fixiert. Die Reinigung über eine ETI-Adsorbersäule hat den Vorteil, daß das ETI-Adsorber-Säulenmaterial direkt aus dem konzentrierten Reoxidationsansatz sogar in Gegenwart von so hohen Argininkonzentrationen wie 0,8 mol/l Arginin beladen werden kann. Eine Aggregation von p-t-PA, wie sie bei niedrigen Argininkonzentrationen unter 25 mmol/l stattfinden kann, wird dadurch vermieden. Besonders bevorzugt erfolgt daher die Reinigung des p-t-PA-Präparates über eine ETI-Adsorbersäule in Gegenwart von 0,2 bis 1,0 M, vorzugsweise 0,5 bis 1,0 M Arginin. Die K1K2P/Pro enthaltende Lösung hat dabei bevorzugt einen pH von mehr als 6,5, besonders bevorzugt mehr als 7.

Die Elution von der ETI-Säule erfolgt durch pH-Erniedrigung in Gegenwart von Arginin unter Bedingungen, die eine gute Löslichkeit von t-PA, das in Prokaryonten exprimiert wurde, ermöglichen. Vorzugsweise liegt der pH bei der Elution im sauren Bereich, besonders bevorzugt im Bereich von 4 bis 6.

Ein erfindungsgemäß hergestelltes K1K2P/Pro-Präparat besitzt eine spezifische t-PA-Aktivität von $\geq$ $0,4 \cdot 10^6$ IU/mg, bevorzugt $\geq 0,6 \cdot 10^6$ IU/mg, dessen Stimulierbarkeit durch Fibrinogenspaltprodukte (Aktivität in Gegenwart von Fibrinogenpeptiden/Aktivität ohne Fibrinogenpeptide) größer als 10fach, bevorzugt größer als 20fach ist. Die Reinheit des erfindungsgemäßen Präparats, beträgt mehr als 90% und bevorzugt mehr als 95%, insbesondere mehr als 98%.

Das erfindungsgemäße t-PA-Derivat ist daher besonders zur Verwendung in einem Arzneimittel zur Gerinnselauflösung geeignet, was wiederum ein weiterer Gegenstand der Erfindung ist.

Das folgende Beispiel erläutert die Erfindung in Verbindung mit der Abbildung weiter.

Fig. 1 zeigt schematisch die Herstellung des Plasmids pA33.

Fig. 2 zeigt den zeitlichen Verlauf der t-PA-Aktivitäts-Plasma-Konzentrationnach intravenöser Bolusinfektion von kommerziell erhältlichem t-PA (ACTILYSE®) im Vergleich zu dem erfindungsgemäßen t-PA-Derivat in linearer Darstellung und

Fig. 3 zeigt den Konzentrationsverlauf in logarithmischer Darstellung.

**Beispiel 1**

Konstruktion des Plasmids pA33.

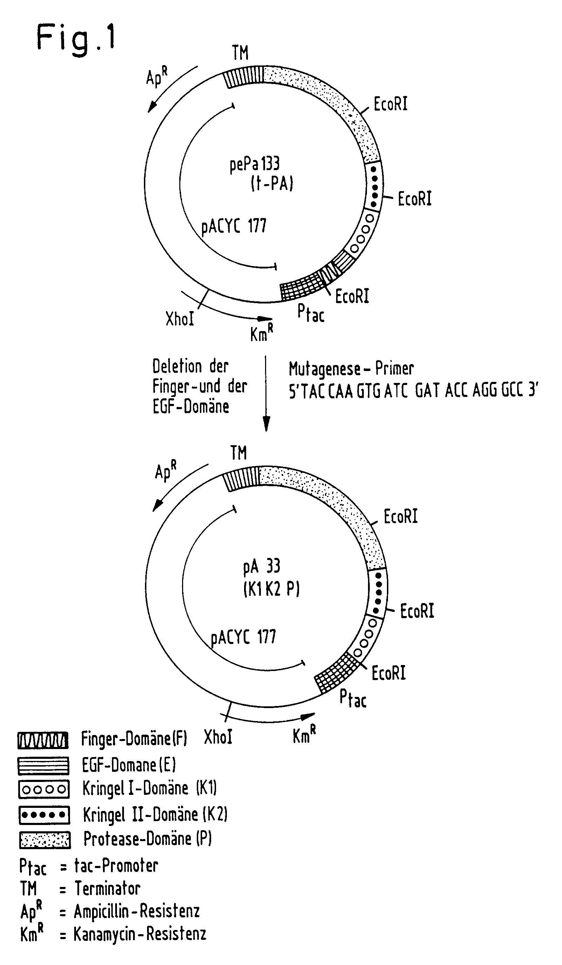

Als Ausgangsplasmid diente das Plasmid pePa 133, das in der Anmeldung EPA 0 242 836 beschrieben ist. Alle experimentellen Schritte zur spezifischen Mutagenese, d.h. zur Deletion von den Domänen F und E aus pePa 133 (siehe Fig. 1) wurden, im wesentlichen unter Anwendung der Methode von Morinaga et al., Biotechnologie 21 (1984), 634 durchgeführt. Es wurden hierzu von pePa 133 zwei Spaltansätze hergestellt. Ansatz A wurde mit EcoR1 gespalten und das größte Fragment isoliert. Ansatz B wurde mit XhoI gespalten und so das Produkt linearisiert. Zur Heteroduplexbildung wurde das folgende Oligonukleotid eingesetzt:

5′     TAC CAA GTG ATC GAT ACC AGG GCC     3′

Durch Koloniehybridisierung mit dem obengenannten Mutagenese-Oligonukleotid wurden diejenigen Klone ermittelt, die das gesuchte Plasmid pA33 enthielten. Dieses Plasmid wurde durch Restriktionsanalyse charakterisiert und auf das Fehlen der SspI-Restriktionsschnittstelle überprüft, die in dem für die Fingerdomäne kodierenden Teil der t-PA-Expressionskassette liegt.

## Beispiel 2

### Präparation von aktivem K1K2P/Pro aus E. Coli

a) Zellyse und Präparation der inclusion bodies (IBs)

1,6 kg Zellfeuchtmasse (E.coli, DSM 3689), transformiert mit dem Plasmid pA33, wurden in 10 l O,1 mol/l Tris-HCL, 20 mmol/l EDTA, pH 6,5, 4°C suspendiert. Dazu wurden 2,5 g Lysozym zugegeben und 30 Minuten bei 4°C inkubiert; anschließend wurde vollständiger Zellaufschluß mittels Hochdruckdispersion durchgeführt. Zur Aufschlußlösung wurden 5 l O,1 mol/l Tris-HCl, 20 mmol/l EDTA, 6 % Triton X100 und 1,5 mol/l NaCl, pH 6,5 zugemischt und weitere 30 Minuten bei 4°C inkubiert. Daran anschließend folgte die Abtrennung der unlöslichen Bestandteile (IB's) durch Zentrifugation.

Das Pellet wurde in 10 l 0,1 mol/l Tris-HCl, 20 mmol/l EDTA, pH 6,5 suspendiert, 30 Minuten bei 4°C inkubiert und IB-Präparat durch anschließende Zentrifugation isoliert.

b) Solubilisierung der IBs

8.7 g IBs (Naßgewicht) wurden in 100 ml 0.1 M Tris, 6 M Guanindin, 0.1 M DTE, pH 7.5 suspendiert und 30 min bei 25 °C gerührt.

Nach Einstellen des pH-Wertes auf pH 3 mit HCl (25%) wurde eine Dialyse gegen 4 mol/l Guaninidin-HCl, pH 2.5 (3 x 3 l, 24 h, 4°C) durchgeführt.

c) Derivatisierung

Das oben genannte Dialysat wurde mit oxidiertem Glutathion (GSSG) ad 50 mmol/l und mit Tris ad 100 mmol/l eingestellt und der pH-Wert mit 5 mol/l NaOH auf 7.5 titriert. Der Ansatz wurde 90 min bei 25 °C inkubiert. Nach Einstellung des pH-Wertes auf pH 3 mit HCl (25%) wurde eine Dialyse gegen 10 mmol/l HCl (3 x 100 l, 48 h, 4 °C) durchgeführt.

d) Naturierung

Ein 10-1-Reaktionsgefäß wurde mit 0,8 mol/l Arg/HCl, pH 8,5, 1 mmol/l EDTA, 1 mmol/l GSH (Glutathion) gefüllt. Die Naturierung wurde bei 20 °C durch 3-fache Zugabe von 100 ml des zuvor gegen 6 mol/l Guanidin-HCl, pH 2.5 dialysierten Derivats im Zeitabstand von 24 Stunden durchgeführt.

Nach der Naturierung erhält man ein Material mit einer spezifischen Aktivität von 50 - 75 KU/mg (Test nach H. Lill, Z. gesamte inn. Med. ihre Grenzgeb. (1987), 42, 478-486).

e) Konzentrierung des Renaturierungsansatzes

Der Renaturierungsansatz kann bei Bedarf über einen Hemodialysator konzentriert werden.

## Beispiel 3

Reinigung von K1K2P/Pro aus E. Coli

Die Reinigung von K1K2P/Pro aus E. Coli erfolgt durch Affinitätschromatographie an Erythrina-Trypsin-Inhibitor (ETI)-Sepharose.

Der Renaturierungsansatz wurde über einen Hemodialysator (Asahi AM 300) 1 : 5 konzentriert, über Nacht gegen 0,8 mol/l Arg/HCl, pH 7,5 dialysiert und zentrifugiert. Eine mit 0,8 mol/l Arg/HCl, pH 7,5 äquilibrierte ETI-Sepharose-Säule (120 ml) wurde mit 1,8 l Dialysat beladen (4 Säulenvolumen (SV)/h) und solange mit Puffer (0.8 mol/l Arg/HCl, pH 7.5, 0.5 mol/l NaCl) gewaschen bis die Absorption des Eluats bei 280 nm den Leerwert des Puffers erreichte. Nach einer 2. Waschung mit 5 SV 0.3 Mol/l Arg/HCl, pH 7.0 erfolgte die Elution mit 0.3 Mol/l Arg/HCl, pH 4.5.

| | Volumen ml | Aktivität KU/ml | $C_{Prot.}$ mg/ml | SA KU/mg | F* |
|---|---|---|---|---|---|
| Dialysat | 1800 | 74 | 1.05 | 70 | 15 |
| K1K2P-Pool | 200 | 610 | 0.82 | 744 | 28 |

* F = Stimulation durch Fibrin = Aktivität in Gegenwart von Fibrin/Aktivität ohne Fibrin.

**Beispiel 4**

Charakterisierung von gereinigtem K1K2P/Pro aus E. coli

a) Proteinchemische Charakterisierung
- SDS-PAGE und Reverse Phase HPLC
  Die Homogenität des über Affinitätschromatographie an ETI-Sepharose gereinigten Materials wurde durch SDS-PAGE und durch Reverse Phase-HPLC (RP-HPLC) gezeigt. Nach elektrophoretischer Analyse des gereinigten Materials errechnete sich aus der relativen Laufstrecke für K1K2P aus E. coli ein apparentes Molekulargewicht von 50.800 Da ± 2.000 Da. Die densitometrische Auswertung ergab eine Reinheit von > 90%.
  Die RP-HPLC beruht auf der unterschiedlichen Wechselwirkung von Proteinen mit hydrophoben Matrices. Diese Eigenschaft wurde als analytische Methode zur Quantifizierung des Reinheitsgrades angewandt.
  Die Analyse des gereinigten K1K2P/Pro aus E. coli erfolgte über eine Nucleosil 300-Trennsäule (Knauer) mit Hilfe eines Trifluoressigsäure/Acetonitril-Gradienten (Puffer A: 1,2 ml Trifluoressigsäure in 1000 ml $H_2O$; Puffer B: 300 ml $H_2O$, 700 ml Acetonitril, 1 ml Trifluoressigsäure; 0 - 100 %). Die Integration der chromatographischen Analyse ergab eine Reinheit von > 95 % .
- N-terminale Sequenz
  Die N-terminale Aminosäuresequenz wurde mit einem ABI 470 Sequenator mit Standard-programm und on-line PTH-Detektion bestimmt. Die gefundene Sequenz S1-Y2-Q3-V4-I5-D6-T7-R8-A9-T10-C11 -Y12-E13-D14 stimmt mit der aus der DNA-Sequenz abgeleiteten, zu erwartenden Sequenz überein.

b) Aktivitätsbestimmung
Die in vitro-Aktivität von K1K2P/Pro aus E. coli wurde nach H. Lill, Z. gesamte inn. Med. ihre Grenzgeb. (1987), 42, 478-486 bestimmt. Die spezifische Aktivität (SA) betrug 650.000 IU/mg ± 200.000 IU/mg. Die Stimulierbarkeit von K1K2P/Pro aus E. coli durch BrCN-Fragmente des Fibrinogens (Aktivität in Gegenwart von Fibrinogen-Fragmenten geteilt durch Aktivität ohne Fibrinogenfragmente) war in diesem Testsystem 25-30.

c) In vitro Bindung an Fibrin
Die in vitro-Bindung von K1K2P/Pro an Fibrin wurde nach der von Higgins und Vehar beschriebenen Methode bestimmt (Higgins, D.L. und Vehar, G.A. (1987), Biochem. 26, 7786-7791).
Dabei zeigt sich, daß K1K2P/pro im Gegensatz zu tPA aus CHO-Zellen keine nennenswerte Fibrinbindung aufweist.

**Beispiel 5**

**Pharmakokinetik von K1K2P/Pro am Kaninchen**

K1K2P/Pro wurde an weißen Neuseeland-Kaninchen mit Actilyse[R] (Alteplase, Thomae GmbH, Biberach, FRG) in seinen pharmakokinetischen Eigenschaften verglichen. Beide Fibrinolytika wurden in einer Dosis von 200 000 IU/kg Körpergewicht (KG) über 1 min intravenös injiziert. Plasmaproben wurden vor und zu definierten Zeitpunkten nach der Injektion gewonnen. Die t-PA-Aktivität im Plasma wurde mit einem spektrophotometrischen Test nach J.H. Verheijen et al. (Thromb. Haemostas. 48, 266, 1982), modifiziert nach H. Lill (Z. Ges. Inn. Med. 42, 478, 1987), gemessen.
Zur Berechnung pharmakokinetischer Parameter wurde ein Computerprogramm der nicht-linearen Regression benutzt, modifiziert nach H.Y. Huang (Aero-Astronautics Report 64, Rice University 1-30, 1969). Bei der Kurvenanpassung wurde individuell unterschiedlich ein 1- oder 2-Kompartimentmodell unterstellt. Vor der Berechnung wurde jeweils der Wert des körpereigenen Basalspiegels von den nachfolgenden Werten abgezogen.
K1K2P/Pro wird nur bei 2 von 6 Tieren mit einer schnellen $\alpha$- und einer langsamen ß-Phase eliminiert. Bei diesen 2 Tieren beträgt der alpha-Phasenanteil an der Gesamtelimination 53 %. Dagegen weisen alle Tiere in der Actilyse[R]-Gruppe eine schnelle alpha-Phase auf, deren Anteil an der Gesamtelimination mehr als 70 % ausmacht. K1K2P/Pro wird vorwiegend mit nur einer Halbwertszeit eliminiert, die 15,1 ± 3,1 min beträgt. Actilyse[R] weist dagegen eine schnelle Halbwertszeit von 1,63 min und eine langsame Halbwertszeit von 10,1 min auf (Tab. 1). Die Gesamtplasma-Clearance von K1K2P/Pro beträgt 6,3 ± 1,5 ml/kg/min und ist damit wesentlich niedriger als bei Actilyse[R] ($Cl_{tot}$ = 22,9 ± 9,1 ml/kg/min).

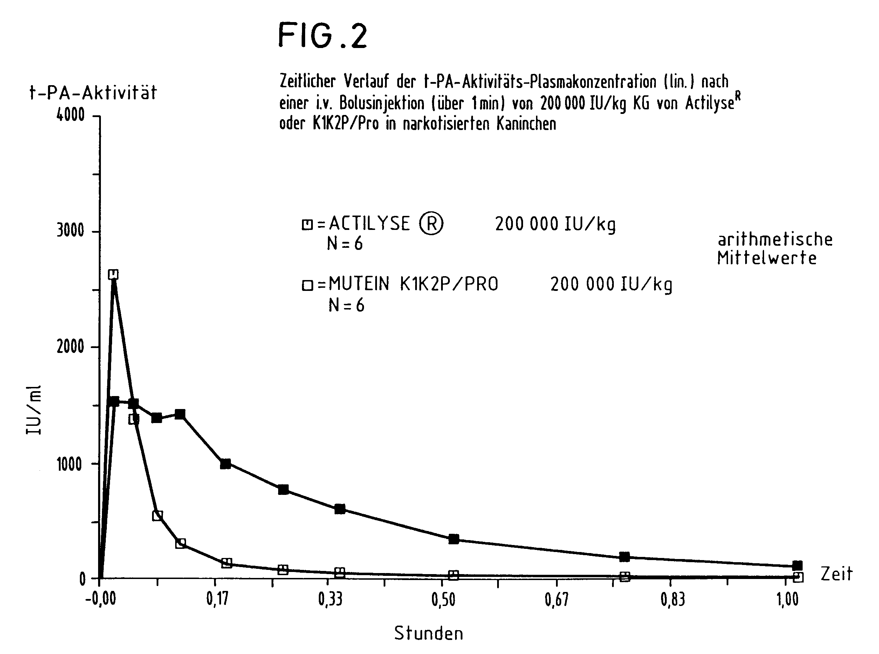

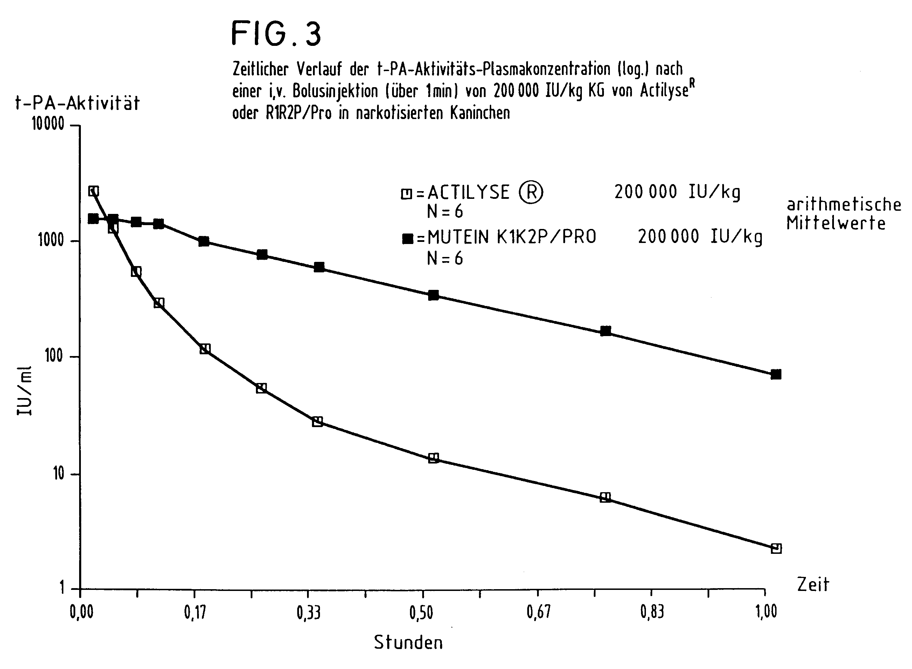

Insgesamt stellt sich K1K2P/Pro als eine t-PA-Mutante dar, die im Vergleich mit Actilyse[R] als dem Stand der Technik ein deutlich verbessertes pharmakokinetisches Profil aufweist (Fig. 2 und 3).

**Beispiel 6**

**Pharmakodynamik von K1K2P/Pro am Kaninchen**

Zur Prüfung der thrombolytischen Effizienz wurde das von D. Collen et al. etablierte Kaninchenmodell der Jugularvenenthrombose angewandt (J. Clin. Invest. 71, 368, 1983). Die Fibrinolytika bzw. das Lösungsmittel wurden intravenös über 1 min als Bolus injiziert. Anschließend wurde die Thrombolyserate ermittelt und ausgewählte Parameter des Gerinnungssystems sowie die Thrombozytenzahl bestimmt (Tab. 2).

Bei gleicher Dosis (200 000 IU/kg KG) erzielte K1K2P/Pro nach intravenöser Bolusinjektion eine statistisch signifikant höhere Thrombolyserate von 50,7 ± 6,5 % als Actilyse[R] (24,1 ± 3,7 %). Die in der Thrombolyse-Effizienz mit K1K2P/Pro vergleichbare Dosis von Actilyse[R] beträgt 800 000 IU/kg KG (Tab. 2).

Bei äquipotenter Dosierung von K1K2P/Pro im Vergleich mit Actilyse[R] ergeben sich gegenüber der Lösungsmittelgruppe geringe Auswirkungen auf die Gerinnungsparameter Fibrinogen, Plasminogen und $alpha_2$-Antiplasmin, die sich jedoch nicht von den Auswirkungen einer äquipotenten Dosis von Actilyse[R] unterscheiden.

K1K2P/Pro ist somit eine t-PA-Mutante, die am Kaninchenmodell der Jugularvenenthrombose nach Bolusinjektion eine thrombolytische Effizienz aufweist, die im Vergleich mit Actilyse[R] wesentlich gesteigert ist. K1K2P/Pro hat dabei seine Fibrinspezifität in einem Ausmaß erhalten, wie sie auch für Actilyse[R] zutrifft.

T a b e l l e    1

Pharmakokinetische Parameter, abgeleitet aus Computerberechnungen von
Plasmakonzentrations-Zeit-Daten auf Basis der t-PA-Aktivität an narkotisierten
Kaninchen nach i.v. Bolusinjektion von 200 000 IU/kg KG
K1K2P/Pro oder Actilyse[R]

| Fibrinolytikum | $t_{1/2\alpha}$ (min) | $t_{1/2\beta}$ (min) | $C_{inj.}$ (IU/ml) | $V_c$ * (ml/kg) | $Cl_{tot}$ * $(ml \times kg^{-1} \times min^{-1})$ | $AUC_{extrapol.}$ * $(IU \times ml^{-1} \times h)$ |
|---|---|---|---|---|---|---|
| Actilyse[R] (n=6) | 1,63 | 10,1 (n=5) | 3051,9 ±1318,8 | 63,2 ±29,3 | 22,9 ±9,1 | 161,1 ± 49,8 |
| K1K2P/Pro (n=6) | 8,9 (n=2) | 15,1 ±3,1 | 1695,9 ± 345,4 | 119,1 ±26,6 | 6,3 ±1,5 | 556,4 ±118,8 |

Mittelwert ± Standardabweichung
*$V_c$ = Verteilungsvolumen des zentralen Kompartiments
$Cl_{tot}$ = Gesamt Plasma-Clearance
AUC = area under the curve; für die Anwendung eines Fibrinolytikums zur Bolusinjektion ist die area under the curve ("AUC") besonders interessant, da sie einen Vergleich der über die Zeit im Plasma herrschenden Konzentrationen zuläßt.

Tabelle 2

**Thrombolyserate, Plättchenzahl und ausgewählte Parameter des Gerinnungssystems nach i.v. Bolusinjektion (über 1 min) von Actilyse[R], K1K2P/Pro oder Lösungsmittel am Kaninchenmodell der Jugularvenenthrombose**

|  | Lösungsmittel | Actilyse[R] 200 000 IU/kg KG | K1K2P/Pro 200 000 IU/kg KG | Actilyse[R] 800 000 IU/kg KG |
|---|---|---|---|---|
| Thrombolyserate(%) | 12,9 ± 0,9 | 24,1 ± 3,7 | 50,7 ± 6,5 | 44,6 ± 4,8 |
| Fibrinogen (%) | 89,5 ± 2,1 | 90,5 ± 2,6 | 81,1 ± 5,1 | 81,4 ± 3,4 |
| Plasminogen (%) | 90,5 ± 2,7 | 83,8 ± 4,4 | 60,1 ± 4,3 | 69,6 ± 3,3 |
| $\alpha_2$-Antiplasmin (%) | 90,9 ± 4,4 | 100,8 ± 5,3 | 67,6 ± 6,7 | 74,2 ± 5,9 |
| PTT (%) | 113,8 ± 4,9 | 112,9 ± 4,3 | 113,1 ± 3,0 | 115,1 ± 5,5 |
| Thrombozyten (%) | 86,1 ±12,1 | 91,4 ± 4,9 | 84,4 ± 8,3 | 86,3 ± 2,8 |

Gerinnungsparameter und Thrombozytenzahl als % des Ausgangswertes vor Injektion
PTT = partielle Thromboplastinzeit
Mittelwert ± SEM        jede Gruppe n = 6 Tiere

**Beispiel 7**

Optimierte Expression in E. coli

Zur Steigerung der Expressionsausbeute wurde die für das K1K2P-Gen kodierende Sequenz in ein Plasmid hoher Kopienzahl umkloniert. Dazu wurde das in der Patentanmeldung P 39 31 933.4 beschriebene Plasmid pePa 126.1 benutzt. Dieses Plasmid setzt sich im wesentlichen aus dem Vektor pKK223-3 und der kodierenden Sequenz für t-PA zusammen, wie sie in der Anmeldung EP-A-0 242 835 beschrieben steht.

In dieses Plasmid wurde zunächst die Sequenz eines fd-Terminators integriert. Dazu wurde das Plasmid pePa 126.1 mit dem Restriktionsenzym Hind III linearisiert. Das so gespaltene Plasmid wurde gelelektrophoretisch aufgetrennt und präparativ gewonnen. Das Plasmid pLBU1 (Gentz et al. (1981) PNAS 78(8):4963) wurde mit Hind III restringiert und ein etwa 360 bp großes Hind III-Fragment, das den fd-Terminator enthält, durch Gelelektrophorese und Gel-Elution präparativ dargestellt. Das linearisierte Plasmid pePa 126.1 und das 360 bp Hind III-Fragment aus pLBU1 wurde ligiert. Der Ligationsansatz wurde mit dem in der Anmeldung P 39 31 933.4 beschriebenen Plasmid pUBS 500 in E. coli, DSM 2102, kotransformiert. Aus den Klonen wurden diejenigen ausgewählt, die das gewünschte Plasmid pePa 126 fd enthalten, das sich vom Ausgangsplasmid pePa 126.1 dadurch unterscheidet, daß es eine zweite Hind III-Schnittstelle enthält.

Aus dem Plasmid pePa 126 fd wurden zwei Fragmente gewonnen: ein 3,4 kb großes BamHI/PvuI-Fragment und ein etwa 1,3 kb großes PvuI/XmaI-Fragment. Die beiden genannten Fragmente wurden mit einem etwa 1,6 kb großen BamHI/XmaI-Fragment aus dem Plasmid pA33 ligiert und mit dem Plasmid pUBS 500 in E. coli transformiert. Das resultierende Plasmid erhielt den Namen pA33 fd und kann von pePa 126 fd dadurch unterschieden werden, daß bei einem Restriktionsansatz mit EcoRI das zweitkleinste EcoRI-Fragment aus pePa 126 fd von etwa 610 bp Länge in pA33 fd um etwa 250 bp verkürzt vorliegt.

**Patentansprüche**
**Patentansprüche für folgende Vertragsstaaten : AT, BE, CH, LI, DE, DK, FR, GB, GR, IT, LU, NL, SE**

**1.** Gewebs-Plasminogen-Aktivator (t-PA)-Derivat, **dadurch gekennzeichnet,** daß es nicht glykosyliert ist und die folgende Aminosäuresequenz aufweist:

(M)

Ser Tyr Gln Val Ile Asp Thr Arg Ala Thr Cys Tyr Glu Asp Gln Gly
                      5                   10                      15

Ile Ser Tyr Arg Gly Thr Trp Ser Thr Ala Glu Ser Gly Ala Glu Cys
                  20                  25                  30

Thr Asn Trp Asn Ser Ser Ala Leu Ala Gln Lys Pro Tyr Ser Gly Arg
              35                  40                  45

Arg Pro Asp Ala Ile Arg Leu Gly Leu Gly Asn His Asn Tyr Cys Arg
          50                  55                  60

Asn Pro Asp Arg Asp Ser Lys Pro Trp Cys Tyr Val Phe Lys Ala Gly
    65                  70                  75                  80

Lys Tyr Ser Ser Glu Phe Cys Ser Thr Pro Ala Cys Ser Glu Gly Asn
                  85                  90                  95

Ser Asp Cys Tyr Phe Gly Asn Gly Ser Ala Tyr Arg Gly Thr His Ser
              100                 105                 110

Leu Thr Glu Ser Gly Ala Ser Cys Leu Pro Trp Asn Ser Met Ile Leu
          115                 120                 125

Ile Gly Lys Val Tyr Thr Ala Gln Asn Pro Ser Ala Gln Ala Leu Gly
    130                 135                 140

Leu Gly Lys His Asn Tyr Cys Arg Asn Pro Asp Gly Asp Ala Lys Pro
145                 150                 155                 160

Trp Cys His Val Leu Lys Asn Arg Arg Leu Thr Trp Glu Tyr Cys Asp
              165                 170                 175

Val Pro Ser Cys Ser Thr Cys Gly Leu Arg Gln Tyr Ser Gln Pro Gln
              180                 185                 190

Phe Arg Ile Lys Gly Gly Leu Phe Ala Asp Ile Ala Ser His Pro Trp
        195                 200                 205

Gln Ala Ala Ile Phe Ala Lys His Arg Arg Ser Pro Gly Glu Arg Phe
        210                 215                 220

Leu Cys Gly Gly Ile Leu Ile Ser Ser Cys Trp Ile Leu Ser Ala Ala
225                 230                 235                 240

```
His Cys Phe Gln Glu Arg Phe Pro Pro His His Leu Thr Val Ile Leu
                245                 250                 255

Gly Arg Thr Tyr Arg Val Val Pro Gly Glu Glu Glu Gln Lys Phe Glu
            260                 265                 270

Val Glu Lys Tyr Ile Val His Lys Glu Phe Asp Asp Asp Thr Tyr Asp
        275                 280                 285

Asn Asp Ile Ala Leu Leu Gln Leu Lys Ser Asp Ser Ser Arg Cys Ala
    290                 295                 300

Gln Glu Ser Ser Val Val Arg Thr Val Cys Leu Pro Pro Ala Asp Leu
305                 310                 315                 320

Gln Leu Pro Asp Trp Thr Glu Cys Glu Leu Ser Gly Tyr Gly Lys His
            325                 330                 335

Glu Ala Leu Ser Pro Phe Tyr Ser Glu Arg Leu Lys Glu Ala His Val
            340                 345                 350

Arg Leu Tyr Pro Ser Ser Arg Cys Thr Ser Gln His Leu Leu Asn Arg
        355                 360                 365

Thr Val Thr Asp Asn Met Leu Cys Ala Gly Asp Thr Arg Ser Gly Gly
    370                 375                 380

Pro Gln Ala Asn Leu His Asp Ala Cys Gln Gly Asp Ser Gly Gly Pro
385                 390                 395                 400

Leu Val Cys Leu Asn Asp Gly Arg Met Thr Leu Val Gly Ile Ile Ser
            405                 410                 415

Trp Gly Leu Gly Cys Gly Gln Lys Asp Val Pro Gly Val Tyr Thr Lys
            420                 425                 430

Val Thr Asn Tyr Leu Asp Trp Ile Arg Asp Asn Met Arg Pro
            435                 440                 445
```

**2.** DNA-Sequenz, **dadurch gekennzeichnet,** daß sie für ein t-PA-Derivat nach Anspruch 1 kodiert und die folgende Sequenz enthält:

```
ATGTCTTACCAAGTGATCGATACCAGGGCCACGTGCTACGAGGACCAGGGCATCAGCTAC
AGGGGCACGTGGAGCACAGCGGAGAGTGGCGCCGAGTGCACCAACTGGAACAGCAGCGCG
TTGGCCCAGAAGCCCTACAGCGGGCGGAGGCCAGACGCCATCAGGCTGGGCCTGGGGAAC
CACAACTACTGCAGAAACCCAGATCGAGACTCAAAGCCCTGGTGCTACGTCTTTAAGGCG
GGGAAGTACAGCTCAGAGTTCTGCAGCACCCCTGCCTGCTCTGAGGGAAACAGTGACTGC
TACTTTGGGAATGGGTCAGCCTACCGTGGCACGCACAGCCTCACCGAGTCGGGTGCCTCC
TGCCTCCCGTGGAATTCCATGATCCTGATAGGCAAGGTTTACACAGCACAGAACCCCAGT
GCCCAGGCACTGGGCCTGGGCAAACATAATTACTGCCGGAATCCTGATGGGGATGCCAAG
CCCTGGTGCCACGTGCTGAAGAACCGCAGGCTGACGTGGGAGTACTGTGATGTGCCCTCC
TGCTCCACCTGCGGCCTGAGACAGTACAGCCAGCCTCAGTTTCGCATCAAAGGAGGGCTC
TTCGCCGACATCGCCTCCCACCCCTGGCAGGCTGCCATCTTTGCCAAGCACAGGAGGTCG
CCCGGAGAGCGGTTCCTGTGCGGGGGCATACTCATCAGCTCCTGCTGGATTCTCTCTGCC
GCCCACTGCTTCCAGGAGAGGTTTCCGCCCCACCACCTGACGGTGATCTTGGGCAGAACA
TACCGGGTGGTCCCTGGCGAGGAGGAGCAGAAATTTGAAGTCGAAAAATACATTGTCCAT
AAGGAATTCGATGATGACACTTACGACAATGACATTGCGCTGCTGCAGCTGAAATCGGAT
TCGTCCCGCTGTGCCCAGGAGAGCAGCGTGGTCCGCACTGTGTGCCTTCCCCCGGCGGAC
CTGCAGCTGCCGGACTGGACGGAGTGTGAGCTCTCCGGCTACGGCAAGCATGAGGCCTTG
TCTCCTTTCTATTCGGAGCGGCTGAAGGAGGCTCATGTCAGACTGTACCCATCCAGCCGC
TGCACATCACAACATTTACTTAACAGAACAGTCACCGACAACATGCTGTGTGCTGGAGAC
ACTCGGAGCGGCGGGCCCCAGGCAAACTTGCACGACGCCTGCCAGGGCGATTCGGGAGGC
CCCCTGGTGTGTCTGAACGATGGCCGCATGACTTTGGTGGGCATCATCAGCTGGGGCCTG
GGCTGTGGACAGAAGGATGTCCCGGGTGTGTACACAAAGGTTACCAACTACCTAGACTGG
ATTCGTGACAACATGCGACCG    1341
```

3. Expressionsplasmid, **dadurch gekennzeichnet,** daß es die DNA-Sequenz nach Anspruch 2, oder eine im Rahmen der Degeneration des genetischen Codes davon verschiedene DNA-Sequenz, die für ein Protein gemäß Anspruch 1 kodiert, enthält.

4. Plasmid pA33 gemäß Fig. 1

5. Verfahren zur Herstellung eines Expressionsplasmids nach einem der Ansprüche 3 oder 4, **dadurch gekennzeichnet,** daß man eine DNA-Sequenz, die für das gesamte t-PA-Protein oder für ein Derivat davon, welches über die Kringel I-, Kringel II- und die Protease-Domänen hinaus noch weitere Bereiche des t-PA-Proteins aufweist, kodiert, in ein Plasmid einbringt und über gerichtete Mutagenese diejenigen Bereiche entfernt, die für Aminosäuren kodieren, die in dem t-PA-Derivat nach Anspruch 1 nicht vorhanden sind.

6. Verfahren nach Anspruch 5, **dadurch gekennzeichnet,** daß man als DNA-Sequenz für das t-PA-Protein oder ein Derivat davon die entsprechende cDNA verwendet.

7. Verfahren zur Herstellung eines t-PA-Derivats nach Anspruch 1, **dadurch gekennzeichnet,** daß man ein Plasmid nach einem der Ansprüche 3 oder 4 in geeignete Wirtszellen einbringt und das Expressionsprodukt aus dem Kulturmedium oder nach Aufschluß der Wirtszellen gewinnt.

EP 0 400 545 B1

**8.** Verfahren nach Anspruch 7, **dadurch gekennzeichnet,** daß man als Wirtszellen Prokaryontenzellen, insbesondere E. coli, verwendet.

**9.** Verfahren nach Anspruch 8, **dadurch gekennzeichnet,** daß man zur Erhöhung der Ausbeute an aktivem Protein gebildete "inclusion bodies" abtrennt, diese durch Behandlung mit Guanidinhydrochlorid solubilisiert, anschließend mit oxidiertem Glutathion derivatisiert und zuletzt das t-PA-Derivat durch Zugabe von L-Arginin und GSH renaturiert.

**10.** Verfahren nach einem der Ansprüche 7 bis 9, **dadurch gekennzeichnet**, daß man in Gegenwart von 25 bis 1000 mmol/l L-Arginin arbeitet.

**11.** Verfahren nach einem der Ansprüche 7 bis 10, **dadurch gekennzeichnet**, daß man nach der Renaturierung das t-PA-Derivat im Renaturierungsansatz konzentriert und anschließend eine chromatographische Reinigung mittels Affinitätschromatographie an einer ETI-Adsorbersäule durchführt.

**12.** Verfahren nach Anspruch 11, **dadurch gekennzeichnet**, daß man die chromatographische Reinigung mit dem Konzentrat des Renaturierungsansatzes, das 25 bis 1000 mmol/l, bevorzugt 200 bis 1000 mmol/l L-Arginin enthält, durchführt.

**13.** Verfahren nach Anspruch 12, **dadurch gekennzeichnet**, daß man das zur chromatographischen Reinigung eingesetzte Konzentrat auf einen pH-Wert von mehr als 7,0 einstellt.

**14.** Verfahren nach einem der Ansprüche 11 bis 13, **dadurch gekennzeichnet**, daß man die Elution bei einem pH-Wert von 4 bis 6 durchführt.

**15.** Mittel zur Auflösung von Blutgerinnseln, enthaltend ein t-PA-Derivat nach Anspruch 1.

**Patentansprüche für folgenden Vertragsstaat : ES**

**1.** Verfahren zur Herstellung eines t-PA-Derivats, das nicht glykosyliert ist und die folgende Aminosäuresequenz aufweist:

16

(M)

Ser Tyr Gln Val Ile Asp Thr Arg Ala Thr Cys Tyr Glu Asp Gln Gly
                    5                   10                  15

Ile Ser Tyr Arg Gly Thr Trp Ser Thr Ala Glu Ser Gly Ala Glu Cys
                20                  25                  30

Thr Asn Trp Asn Ser Ser Ala Leu Ala Gln Lys Pro Tyr Ser Gly Arg
            35                  40                  45

Arg Pro Asp Ala Ile Arg Leu Gly Leu Gly Asn His Asn Tyr Cys Arg
        50                  55                  60

Asn Pro Asp Arg Asp Ser Lys Pro Trp Cys Tyr Val Phe Lys Ala Gly
    65                  70                  75                  80

Lys Tyr Ser Ser Glu Phe Cys Ser Thr Pro Ala Cys Ser Glu Gly Asn
                85                  90                  95

Ser Asp Cys Tyr Phe Gly Asn Gly Ser Ala Tyr Arg Gly Thr His Ser
            100                 105                 110

Leu Thr Glu Ser Gly Ala Ser Cys Leu Pro Trp Asn Ser Met Ile Leu
        115                 120                 125

Ile Gly Lys Val Tyr Thr Ala Gln Asn Pro Ser Ala Gln Ala Leu Gly
    130                 135                 140

Leu Gly Lys His Asn Tyr Cys Arg Asn Pro Asp Gly Asp Ala Lys Pro
145                 150                 155                 160

Trp Cys His Val Leu Lys Asn Arg Arg Leu Thr Trp Glu Tyr Cys Asp
            165                 170                 175

Val Pro Ser Cys Ser Thr Cys Gly Leu Arg Gln Tyr Ser Gln Pro Gln
            180                 185                 190

17

```
Phe Arg Ile Lys Gly Gly Leu Phe Ala Asp Ile Ala Ser His Pro Trp
        195                 200             205

Gln Ala Ala Ile Phe Ala Lys His Arg Arg Ser Pro Gly Glu Arg Phe
        210                 215             220

Leu Cys Gly Gly Ile Leu Ile Ser Ser Cys Trp Ile Leu Ser Ala Ala
225                 230             235                 240

His Cys Phe Gln Glu Arg Phe Pro Pro His His Leu Thr Val Ile Leu
                245             250                 255

Gly Arg Thr Tyr Arg Val Val Pro Gly Glu Glu Glu Gln Lys Phe Glu
            260             265             270

Val Glu Lys Tyr Ile Val His Lys Glu Phe Asp Asp Asp Thr Tyr Asp
            275             280             285

Asn Asp Ile Ala Leu Leu Gln Leu Lys Ser Asp Ser Ser Arg Cys Ala
        290             295             300

Gln Glu Ser Ser Val Val Arg Thr Val Cys Leu Pro Pro Ala Asp Leu
305                 310             315                 320

Gln Leu Pro Asp Trp Thr Glu Cys Glu Leu Ser Gly Tyr Gly Lys His
            325             330             335

Glu Ala Leu Ser Pro Phe Tyr Ser Glu Arg Leu Lys Glu Ala His Val
            340             345             350

Arg Leu Tyr Pro Ser Ser Arg Cys Thr Ser Gln His Leu Leu Asn Arg
            355             360             365

Thr Val Thr Asp Asn Met Leu Cys Ala Gly Asp Thr Arg Ser Gly Gly
        370             375             380

Pro Gln Ala Asn Leu His Asp Ala Cys Gln Gly Asp Ser Gly Gly Pro
385                 390             395                 400

Leu Val Cys Leu Asn Asp Gly Arg Met Thr Leu Val Gly Ile Ile Ser
            405             410             415

Trp Gly Leu Gly Cys Gly Gln Lys Asp Val Pro Gly Val Tyr Thr Lys
            420             425             430

Val Thr Asn Tyr Leu Asp Trp Ile Arg Asp Asn Met Arg Pro
            435             440             445
```

**dadurch gekennzeichnet,**
daß man ein Expressionsplasmid, das die DNA-Sequenz

```
ATGTCTTACCAAGTGATCGATACCAGGGCCACGTGCTACGAGGACCAGGGCATCAGCTAC
AGGGGCACGTGGAGCACAGCGGAGAGTGGCGCCGAGTGCACCAACTGGAACAGCAGCGCG
TTGGCCCAGAAGCCCTACAGCGGGCGGAGGCCAGACGCCATCAGGCTGGGCCTGGGGAAC
CACAACTACTGCAGAAACCCAGATCGAGACTCAAAGCCCTGGTGCTACGTCTTTAAGGCG
GGGAAGTACAGCTCAGAGTTCTGCAGCACCCCTGCCTGCTCTGAGGGAAACAGTGACTGC
TACTTTGGGAATGGGTCAGCCTACCGTGGCACGCACAGCCTCACCGAGTCGGGTGCCTCC
TGCCTCCCGTGGAATTCCATGATCCTGATAGGCAAGGTTTACACAGCACAGAACCCCAGT
GCCCAGGCACTGGGCCTGGGCAAACATAATTACTGCCGGAATCCTGATGGGGATGCCAAG
CCCTGGTGCCACGTGCTGAAGAACCGCAGGCTGACGTGGGAGTACTGTGATGTGCCCTCC
TGCTCCACCTGCGGCCTGAGACAGTACAGCCAGCCTCAGTTTCGCATCAAAGGAGGGCTC
TTCGCCGACATCGCCTCCCACCCCTGGCAGGCTGCCATCTTTGCCAAGCACAGGAGGTCG
CCCGGAGAGCGGTTCCTGTGCGGGGGCATACTCATCAGCTCCTGCTGGATTCTCTCTGCC
GCCCACTGCTTCCAGGAGAGGTTTCCGCCCCACCACCTGACGGTGATCTTGGGCAGAACA
TACCGGGTGGTCCCTGGCGAGGAGGAGCAGAAATTTGAAGTCGAAAAATACATTGTCCAT
AAGGAATTCGATGATGACACTTACGACAATGACATTGCGCTGCTGCAGCTGAAATCGGAT
TCGTCCCGCTGTGCCCAGGAGAGCAGCGTGGTCCGCACTGTGTGCCTTCCCCCGGCGGAC
CTGCAGCTGCCGGACTGGACGGAGTGTGAGCTCTCCGGCTACGGCAAGCATGAGGCCTTG
TCTCCTTTCTATTCGGAGCGGCTGAAGGAGGCTCATGTCAGACTGTACCCATCCAGCCGC
TGCACATCACAACATTTACTTAACAGAACAGTCACCGACAACATGCTGTGTGCTGGAGAC
ACTCGGAGCGGCGGGCCCCAGGCAAACTTGCACGACGCCTGCCAGGGCGATTCGGGAGGC
CCCCTGGTGTGTCTGAACGATGGCCGCATGACTTTGGTGGGCATCATCAGCTGGGGCCTG
GGCTGTGGACAGAAGGATGTCCCGGGTGTGTACACAAAGGTTACCAACTACCTAGACTGG
ATTCGTGACAACATGCGACCG   1341
```

oder eine im Rahmen der Degeneration des genetischen Codes davon verschiedene DNA-Sequenz, die für ein entsprechendes t-PA-Derivat kodiert, enthält, in geeignete Wirtszellen einbringt und das Expressionsprodukt aus dem Kulturmedium oder nach Aufschluß der Wirtszellen gewinnt.

2. Verfahren nach Anspruch 1,
   **dadurch gekennzeichnet,**
   daß man das Plasmid pA33 gemäß Figur 1 verwendet.

3. Verfahren nach Anspruch 1 oder 2,
   **dadurch gekennzeichnet,**
   daß man als Wirtszellen Prokaryontenzellen, insbesondere E.coli, verwendet.

4. Verfahren nach Anspruch 1, 2 oder 3,
   **dadurch gekennzeichnet,**
   daß man zur Erhöhung der Ausbeute an aktivem Protein gebildete "inclusion bodies" abtrennt, diese durch Behandlung mit Guanidinhydrochlorid solubilisiert, anschließend mit oxidiertem Glutathion derivatisiert und zuletzt das t-PA-Derivat durch Zugabe von L-Arginin und GSH renaturiert.

5. Verfahren nach einem der Ansprüche 1 bis 4,
   **dadurch gekennzeichnet,**
   daß man in Gegenwart von 25 bis 1000 mmol/l L-Arginin arbeitet.

**6.** Verfahren nach einem der Ansprüche 1 bis 5,
**dadurch gekennzeichnet,**
daß man nach der Renaturierung das t-PA-Derivat im Renaturierungsansatz konzentriert und anschließend eine chromatographische Reinigung mittels Affinitätschromatographie an einer ETI-Adsorbersäule durchführt.

**7.** Verfahren nach Anspruch 6,
**dadurch gekennzeichnet,**
daß man die chromatographische Reinigung mit dem Konzentrat des Renaturierungsansatzes, das 25 bis 1000 mmol/l, bevorzugt 200 bis 1000 mmol/l L-Arginin enthält, durchführt.

**8.** Verfahren nach Anspruch 7,
**dadurch gekennzeichnet,**
daß man das zur chromatographischen Reinigung eingesetzte Konzentrat auf einen pH-Wert von mehr als 7,0 einstellt.

**9.** Verfahren nach einem der Ansprüche 6 bis 8,
**dadurch gekennzeichnet,**
daß man die Elution bei einem pH-Wert von 4 bis 6 durchführt.

**10.** Verfahren zur Herstellung eines Expressionsplasmids, das die DNA-Sequenz

```
ATGTCTTACCAAGTGATCGATACCAGGGCCACGTGCTACGAGGACCAGGGCATCAGCTAC
AGGGGCACGTGGAGCACAGCGGAGAGTGGCGCCGAGTGCACCAACTGGAACAGCAGCGCG
TTGGCCCAGAAGCCCTACAGCGGGCGGAGGCCAGACGCCATCAGGCTGGGCCTGGGGAAC
CACAACTACTGCAGAAACCCAGATCGAGACTCAAAGCCCTGGTGCTACGTCTTTAAGGCG
GGGAAGTACAGCTCAGAGTTCTGCAGCACCCCTGCCTGCTCTGAGGGAAACAGTGACTGC
TACTTTGGGAATGGGTCAGCCTACCGTGGCACGCACAGCCTCACCGAGTCGGGTGCCTCC
TGCCTCCCGTGGAATTCCATGATCCTGATAGGCAAGGTTTACACAGCACAGAACCCCAGT
GCCCAGGCACTGGGCCTGGGCAAACATAATTACTGCCGGAATCCTGATGGGGATGCCAAG
CCCTGGTGCCACGTGCTGAAGAACCGCAGGCTGACGTGGGAGTACTGTGATGTGCCCTCC
TGCTCCACCTGCGGCCTGAGACAGTACAGCCAGCCTCAGTTTCGCATCAAAGGAGGGCTC
TTCGCCGACATCGCCTCCCACCCCTGGCAGGCTGCCATCTTTGCCAAGCACAGGAGGTCG
CCCGGAGAGCGGTTCCTGTGCGGGGGCATACTCATCAGCTCCTGCTGGATTCTCTCTGCC
GCCCACTGCTTCCAGGAGAGGTTTCCGCCCCACCACCTGACGGTGATCTTGGGCAGAACA
TACCGGGTGGTCCCTGGCGAGGAGGAGCAGAAATTTGAAGTCGAAAAATACATTGTCCAT
AAGGAATTCGATGATGACACTTACGACAATGACATTGCGCTGCTGCAGCTGAAATCGGAT
TCGTCCCGCTGTGCCCAGGAGAGCAGCGTGGTCCGCACTGTGTGCCTTCCCCCGGCGGAC
CTGCAGCTGCCGGACTGGACGGAGTGTGAGCTCTCCGGCTACGGCAAGCATGAGGCCTTG
TCTCCTTTCTATTCGGAGCGGCTGAAGGAGGCTCATGTCAGACTGTACCCATCCAGCCGC
TGCACATCACAACATTTACTTAACAGAACAGTCACCGACAACATGCTGTGTGCTGGAGAC
ACTCGGAGCGGCGGGCCCCAGGCAAACTTGCACGACGCCTGCCAGGGCGATTCGGGAGGC
CCCCTGGTGTGTCTGAACGATGGCCGCATGACTTTGGTGGGCATCATCAGCTGGGGCCTG
GGCTGTGGACAGAAGGATGTCCCGGGTGTGTACACAAAGGTTACCAACTACCTAGACTGG
ATTCGTGACAACATGCGACCG    1341
```

oder eine im Rahmen der Degeneration des genetischen Codes davon verschiedene DNA-Sequenz, die für ein Protein kodiert, enthält, das nicht glykosyliert ist und die folgende Aminosäuresequenz aufweist

(M)

```
Ser Tyr Gln Val Ile Asp Thr Arg Ala Thr Cys Tyr Glu Asp Gln Gly
                  5                   10                  15
Ile Ser Tyr Arg Gly Thr Trp Ser Thr Ala Glu Ser Gly Ala Glu Cys
            20                  25                  30
Thr Asn Trp Asn Ser Ser Ala Leu Ala Gln Lys Pro Tyr Ser Gly Arg
            35                  40                  45
Arg Pro Asp Ala Ile Arg Leu Gly Leu Gly Asn His Asn Tyr Cys Arg
        50                  55                  60
Asn Pro Asp Arg Asp Ser Lys Pro Trp Cys Tyr Val Phe Lys Ala Gly
65                  70                  75                  80
Lys Tyr Ser Ser Glu Phe Cys Ser Thr Pro Ala Cys Ser Glu Gly Asn
                85                  90                  95
Ser Asp Cys Tyr Phe Gly Asn Gly Ser Ala Tyr Arg Gly Thr His Ser
            100                 105                 110
Leu Thr Glu Ser Gly Ala Ser Cys Leu Pro Trp Asn Ser Met Ile Leu
        115                 120                 125
Ile Gly Lys Val Tyr Thr Ala Gln Asn Pro Ser Ala Gln Ala Leu Gly
        130                 135                 140
Leu Gly Lys His Asn Tyr Cys Arg Asn Pro Asp Gly Asp Ala Lys Pro
145                 150                 155                 160
Trp Cys His Val Leu Lys Asn Arg Arg Leu Thr Trp Glu Tyr Cys Asp
                165                 170                 175
Val Pro Ser Cys Ser Thr Cys Gly Leu Arg Gln Tyr Ser Gln Pro Gln
            180                 185                 190
Phe Arg Ile Lys Gly Gly Leu Phe Ala Asp Ile Ala Ser His Pro Trp
        195                 200                 205
Gln Ala Ala Ile Phe Ala Lys His Arg Arg Ser Pro Gly Glu Arg Phe
        210                 215                 220
Leu Cys Gly Gly Ile Leu Ile Ser Ser Cys Trp Ile Leu Ser Ala Ala
225                 230                 235                 240
```

21

```
His Cys Phe Gln Glu Arg Phe Pro Pro His His Leu Thr Val Ile Leu
            245             250                 255

Gly Arg Thr Tyr Arg Val Val Pro Gly Glu Glu Glu Gln Lys Phe Glu
            260             265                 270

Val Glu Lys Tyr Ile Val His Lys Glu Phe Asp Asp Asp Thr Tyr Asp
            275             280             285

Asn Asp Ile Ala Leu Leu Gln Leu Lys Ser Asp Ser Ser Arg Cys Ala
    290             295             300

Gln Glu Ser Ser Val Val Arg Thr Val Cys Leu Pro Pro Ala Asp Leu
305             310             315                 320

Gln Leu Pro Asp Trp Thr Glu Cys Glu Leu Ser Gly Tyr Gly Lys His
            325             330                 335

Glu Ala Leu Ser Pro Phe Tyr Ser Glu Arg Leu Lys Glu Ala His Val
            340             345             350

Arg Leu Tyr Pro Ser Ser Arg Cys Thr Ser Gln His Leu Leu Asn Arg
            355             360             365

Thr Val Thr Asp Asn Met Leu Cys Ala Gly Asp Thr Arg Ser Gly Gly
    370             375             380

Pro Gln Ala Asn Leu His Asp Ala Cys Gln Gly Asp Ser Gly Gly Pro
385             390             395                 400

Leu Val Cys Leu Asn Asp Gly Arg Met Thr Leu Val Gly Ile Ile Ser
            405             410                 415

Trp Gly Leu Gly Cys Gly Gln Lys Asp Val Pro Gly Val Tyr Thr Lys
            420             425             430

Val Thr Asn Tyr Leu Asp Trp Ile Arg Asp Asn Met Arg Pro
            435             440             445
```

oder von Plasmid pA33 gemäß Figur 1,

**dadurch gekennzeichnet,**

daß man eine DNA-Sequenz, die für das gesamte t-PA-Protein oder für ein Derivat davon, welches über die Kringel I-, Kringel II- und die Protease-Domänen hinaus noch weitere Bereiche des t-PA-Proteins aufweist, kodiert, in ein Plasmid einbringt und über gerichtete Mutagenese diejenigen Bereiche entfernt, die für Aminosäuren kodieren, die in dem t-PA-Derivat nicht vorhanden sind.

**11.** Verfahren nach Anspruch 10,

**dadurch gekennzeichnet,**

daß man als DNA-Sequenz für das t-PA-Protein oder ein Derivat davon die entsprechende cDNA verwendet.

**Claims**

**Claims for the following Contracting States : AT, BE, CH, LI, DE, DK, FR, GB, GR, IT, LU, NL, SE**

**1.** Derivative of tissue-type plasminogen activator (t-PA),

**wherein**

it is not glycosylated and has the following amino acid sequence:

(M)

Ser Tyr Gln Val Ile Asp Thr Arg Ala Thr Cys Tyr Glu Asp Gln Gly
                      5                   10                  15

Ile Ser Tyr Arg Gly Thr Trp Ser Thr Ala Glu Ser Gly Ala Glu Cys
                 20                  25                  30

Thr Asn Trp Asn Ser Ser Ala Leu Ala Gln Lys Pro Tyr Ser Gly Arg
             35                  40                  45

Arg Pro Asp Ala Ile Arg Leu Gly Leu Gly Asn His Asn Tyr Cys Arg
        50                  55                  60

Asn Pro Asp Arg Asp Ser Lys Pro Trp Cys Tyr Val Phe Lys Ala Gly
    65                  70                  75                  80

Lys Tyr Ser Ser Glu Phe Cys Ser Thr Pro Ala Cys Ser Glu Gly Asn
                 85                  90                  95

Ser Asp Cys Tyr Phe Gly Asn Gly Ser Ala Tyr Arg Gly Thr His Ser
            100                 105                 110

Leu Thr Glu Ser Gly Ala Ser Cys Leu Pro Trp Asn Ser Met Ile Leu
        115                 120                 125

Ile Gly Lys Val Tyr Thr Ala Gln Asn Pro Ser Ala Gln Ala Leu Gly
    130                 135                 140

Leu Gly Lys His Asn Tyr Cys Arg Asn Pro Asp Gly Asp Ala Lys Pro
145                 150                 155                 160

Trp Cys His Val Leu Lys Asn Arg Arg Leu Thr Trp Glu Tyr Cys Asp
            165                 170                 175

Val Pro Ser Cys Ser Thr Cys Gly Leu Arg Gln Tyr Ser Gln Pro Gln
            180                 185                 190

Phe Arg Ile Lys Gly Gly Leu Phe Ala Asp Ile Ala Ser His Pro Trp
        195                 200                 205

Gln Ala Ala Ile Phe Ala Lys His Arg Arg Ser Pro Gly Glu Arg Phe
    210                 215                 220

Leu Cys Gly Gly Ile Leu Ile Ser Ser Cys Trp Ile Leu Ser Ala Ala
225                 230                 235                 240

```
His Cys Phe Gln Glu Arg Phe Pro Pro His His Leu Thr Val Ile Leu
             245                 250                 255
Gly Arg Thr Tyr Arg Val Val Pro Gly Glu Glu Glu Gln Lys Phe Glu
             260                 265                 270
Val Glu Lys Tyr Ile Val His Lys Glu Phe Asp Asp Asp Thr Tyr Asp
         275                 280                 285
Asn Asp Ile Ala Leu Leu Gln Leu Lys Ser Asp Ser Ser Arg Cys Ala
    290                 295                 300
Gln Glu Ser Ser Val Val Arg Thr Val Cys Leu Pro Pro Ala Asp Leu
305                 310                 315                 320
Gln Leu Pro Asp Trp Thr Glu Cys Glu Leu Ser Gly Tyr Gly Lys His
             325                 330                 335
Glu Ala Leu Ser Pro Phe Tyr Ser Glu Arg Leu Lys Glu Ala His Val
             340                 345                 350
Arg Leu Tyr Pro Ser Ser Arg Cys Thr Ser Gln His Leu Leu Asn Arg
         355                 360                 365
Thr Val Thr Asp Asn Met Leu Cys Ala Gly Asp Thr Arg Ser Gly Gly
    370                 375                 380
Pro Gln Ala Asn Leu His Asp Ala Cys Gln Gly Asp Ser Gly Gly Pro
385                 390                 395                 400
Leu Val Cys Leu Asn Asp Gly Arg Met Thr Leu Val Gly Ile Ile Ser
             405                 410                 415
Trp Gly Leu Gly Cys Gly Gln Lys Asp Val Pro Gly Val Tyr Thr Lys
             420                 425                 430
Val Thr Asn Tyr Leu Asp Trp Ile Arg Asp Asn Met Arg Pro
         435                 440                 445
```

2. DNA sequence, **wherein**
it codes for a t-PA derivative as claimed in claim 1 and contains the following sequence:

```
ATGTCTTACCAAGTGATCGATACCAGGGCCACGTGCTACGAGGACCAGGGCATCAGCTAC
AGGGGCACGTGGAGCACAGCGGAGAGTGGCGCCGAGTGCACCAACTGGAACAGCAGCGCG
TTGGCCCAGAAGCCCTACAGCGGGCGGAGGCCAGACGCCATCAGGCTGGGCCTGGGGAAC
CACAACTACTGCAGAAACCCAGATCGAGACTCAAAGCCCTGGTGCTACGTCTTTAAGGCG
GGGAAGTACAGCTCAGAGTTCTGCAGCACCCCTGCCTGCTCTGAGGGAAACAGTGACTGC
TACTTTGGGAATGGGTCAGCCTACCGTGGCACGCACAGCCTCACCGAGTCGGGTGCCTCC
TGCCTCCCGTGGAATTCCATGATCCTGATAGGCAAGGTTTACACAGCACAGAACCCCAGT
GCCCAGGCACTGGGCCTGGGCAAACATAATTACTGCCGGAATCCTGATGGGGATGCCAAG
CCCTGGTGCCACGTGCTGAAGAACCGCAGGCTGACGTGGGAGTACTGTGATGTGCCCTCC
TGCTCCACCTGCGGCCTGAGACAGTACAGCCAGCCTCAGTTTCGCATCAAAGGAGGGCTC
TTCGCCGACATCGCCTCCCACCCCTGGCAGGCTGCCATCTTTGCCAAGCACAGGAGGTCG
CCCGGAGAGCGGTTCCTGTGCGGGGGCATACTCATCAGCTCCTGCTGGATTCTCTCTGCC
GCCCACTGCTTCCAGGAGAGGTTTCCGCCCCACCACCTGACGGTGATCTTGGGCAGAACA
TACCGGGTGGTCCCTGGCGAGGAGGAGCAGAAATTTGAAGTCGAAAAATACATTGTCCAT
AAGGAATTCGATGATGACACTTACGACAATGACATTGCGCTGCTGCAGCTGAAATCGGAT
TCGTCCCGCTGTGCCCAGGAGAGCAGCGTGGTCCGCACTGTGTGCCTTCCCCCGGCGGAC
CTGCAGCTGCCGGACTGGACGGAGTGTGAGCTCTCCGGCTACGGCAAGCATGAGGCCTTG
TCTCCTTTCTATTCGGAGCGGCTGAAGGAGGCTCATGTCAGACTGTACCCATCCAGCCGC
TGCACATCACAACATTTACTTAACAGAACAGTCACCGACAACATGCTGTGTGCTGGAGAC
ACTCGGAGCGGCGGGCCCCAGGCAAACTTGCACGACGCCTGCCAGGGCGATTCGGGAGGC
CCCCTGGTGTGTCTGAACGATGGCCGCATGACTTTGGTGGGCATCATCAGCTGGGGCCTG
GGCTGTGGACAGAAGGATGTCCCGGGTGTGTACACAAAGGTTACCAACTACCTAGACTGG
ATTCGTGACAACATGCGACCG   1341
```

3.  Expression plasmid,
    **wherein**
    it contains the DNA sequence as claimed in claim 2 or a different DNA sequence within the scope of the degeneracy of the genetic code which codes for a protein as claimed in claim 1.

4.  Plasmid pA33 according to Fig. 1.

5.  Process for the construction of an expression plasmid as claimed in one of the claims 3 or 4,
    **wherein**
    a DNA sequence which codes for the whole t-PA protein or for a derivative thereof that contains further regions of the t-PA protein in addition to the kringle I, kringle II and the protease domains is introduced into a plasmid and those domains which code for amino acids that are not present in the t-PA derivative as claimed in claim 1 are removed by site-directed mutagenesis.

6.  Process as claimed in claim 5,
    **wherein**
    the corresponding cDNA is used as the DNA sequence for the t-PA protein or a derivative thereof.

7.  Process for the production of a t-PA derivative as claimed in claim 1,
    **wherein**
    a plasmid as claimed in one of the claims 3 or 4 is introduced into suitable host cells and the

25

expression product is isolated from the culture medium or after lysis of the host cells.

8. Process as claimed in claim 7,
   **wherein**
   prokaryotic cells and in particular E. coli are used as host cells.

9. Process as claimed in claim 8,
   **wherein**
   the yield of active protein is increased by isolating the "inclusion bodies" that form and solubilizing them by treatment with guanidine hydrochloride, followed by derivatization with oxidized glutathione and finally renaturation of the t-PA derivative by addition of L-arginine and GSH.

10. Process as claimed in one of the claims 7 to 9,
    **wherein**
    one works in the presence of 25 to 1000 mmol/l L-arginine.

11. Process as claimed in one of the claims 7 to 10,
    **wherein**
    after the renaturation, the t-PA in the renaturation mixture is concentrated and subsequently a chromatographic purification by means of affinity chromatography on an ETI adsorber column is carried out.

12. Process as claimed in claim 11,
    **wherein**
    the chromatographic purification is carried out with the concentrate of the renaturation mixture which contains 25 to 1000 mmol/l, preferably 200 to 1000 mmol/l L-arginine.

13. Process as claimed in claim 12,
    **wherein**
    the concentrate used for the chromatographic purification is adjusted to a pH value of more than 7.0.

14. Process as claimed in one of the claims 11 to 13,
    **wherein**
    the elution is carried out at a pH value of 4 to 6.

15. Agent for dissolving blood clots containing a t-PA derivative as claimed in claim 1.

**Claims for the following Contracting State : ES**

1. Process for the production of a t-PA derivative which is not glycosylated and has the following amino acid sequence:

(M)

Ser Tyr Gln Val Ile Asp Thr Arg Ala Thr Cys Tyr Glu Asp Gln Gly
                    5                   10                  15

Ile Ser Tyr Arg Gly Thr Trp Ser Thr Ala Glu Ser Gly Ala Glu Cys
                20                  25                  30

Thr Asn Trp Asn Ser Ser Ala Leu Ala Gln Lys Pro Tyr Ser Gly Arg
            35                  40                  45

Arg Pro Asp Ala Ile Arg Leu Gly Leu Gly Asn His Asn Tyr Cys Arg
        50                  55                  60

Asn Pro Asp Arg Asp Ser Lys Pro Trp Cys Tyr Val Phe Lys Ala Gly
    65                  70                  75                  80

Lys Tyr Ser Ser Glu Phe Cys Ser Thr Pro Ala Cys Ser Glu Gly Asn
                85                  90                  95

Ser Asp Cys Tyr Phe Gly Asn Gly Ser Ala Tyr Arg Gly Thr His Ser
            100                 105                 110

Leu Thr Glu Ser Gly Ala Ser Cys Leu Pro Trp Asn Ser Met Ile Leu
        115                 120                 125

Ile Gly Lys Val Tyr Thr Ala Gln Asn Pro Ser Ala Gln Ala Leu Gly
    130                 135                 140

Leu Gly Lys His Asn Tyr Cys Arg Asn Pro Asp Gly Asp Ala Lys Pro
    145                 150                 155                 160

Trp Cys His Val Leu Lys Asn Arg Arg Leu Thr Trp Glu Tyr Cys Asp
                165                 170                 175

Val Pro Ser Cys Ser Thr Cys Gly Leu Arg Gln Tyr Ser Gln Pro Gln
            180                 185                 190

27

```
Phe Arg Ile Lys Gly Gly Leu Phe Ala Asp Ile Ala Ser His Pro Trp
        195             200             205

Gln Ala Ala Ile Phe Ala Lys His Arg Arg Ser Pro Gly Glu Arg Phe
    210             215             220

Leu Cys Gly Gly Ile Leu Ile Ser Ser Cys Trp Ile Leu Ser Ala Ala
225             230             235             240

His Cys Phe Gln Glu Arg Phe Pro Pro His His Leu Thr Val Ile Leu
            245             250             255

Gly Arg Thr Tyr Arg Val Val Pro Gly Glu Glu Glu Gln Lys Phe Glu
            260             265             270

Val Glu Lys Tyr Ile Val His Lys Glu Phe Asp Asp Asp Thr Tyr Asp
        275             280             285

Asn Asp Ile Ala Leu Leu Gln Leu Lys Ser Asp Ser Ser Arg Cys Ala
    290             295             300

Gln Glu Ser Ser Val Val Arg Thr Val Cys Leu Pro Pro Ala Asp Leu
305             310             315             320

Gln Leu Pro Asp Trp Thr Glu Cys Glu Leu Ser Gly Tyr Gly Lys His
            325             330             335

Glu Ala Leu Ser Pro Phe Tyr Ser Glu Arg Leu Lys Glu Ala His Val
            340             345             350

Arg Leu Tyr Pro Ser Ser Arg Cys Thr Ser Gln His Leu Leu Asn Arg
        355             360             365

Thr Val Thr Asp Asn Met Leu Cys Ala Gly Asp Thr Arg Ser Gly Gly
    370             375             380

Pro Gln Ala Asn Leu His Asp Ala Cys Gln Gly Asp Ser Gly Gly Pro
385             390             395             400

Leu Val Cys Leu Asn Asp Gly Arg Met Thr Leu Val Gly Ile Ile Ser
            405             410             415

Trp Gly Leu Gly Cys Gly Gln Lys Asp Val Pro Gly Val Tyr Thr Lys
            420             425             430

Val Thr Asn Tyr Leu Asp Trp Ile Arg Asp Asn Met Arg Pro
        435             440             445
```

**wherein,**

an expression plasmid which contains the DNA sequence

```
ATGTCTTACCAAGTGATCGATACCAGGGCCACGTGCTACGAGGACCAGGGCATCAGCTAC
AGGGGCACGTGGAGCACAGCGGAGAGTGGCGCCGAGTGCACCAACTGGAACAGCAGCGCG
TTGGCCCAGAAGCCCTACAGCGGGCGGAGGCCAGACGCCATCAGGCTGGGCCTGGGGAAC
CACAACTACTGCAGAAACCCAGATCGAGACTCAAAGCCCTGGTGCTACGTCTTTAAGGCG
GGGAAGTACAGCTCAGAGTTCTGCAGCACCCCTGCCTGCTCTGAGGGAAACAGTGACTGC
TACTTTGGGAATGGGTCAGCCTACCGTGGCACGCACAGCCTCACCGAGTCGGGTGCCTCC
TGCCTCCCGTGGAATTCCATGATCCTGATAGGCAAGGTTTACACAGCACAGAACCCCAGT
GCCCAGGCACTGGGCCTGGGCAAACATAATTACTGCCGGAATCCTGATGGGGATGCCAAG
CCCTGGTGCCACGTGCTGAAGAACCGCAGGCTGACGTGGGAGTACTGTGATGTGCCCTCC
TGCTCCACCTGCGGCCTGAGACAGTACAGCCAGCCTCAGTTTCGCATCAAAGGAGGGCTC
TTCGCCGACATCGCCTCCCACCCCTGGCAGGCTGCCATCTTTGCCAAGCACAGGAGGTCG
CCCGGAGAGCGGTTCCTGTGCGGGGGCATACTCATCAGCTCCTGCTGGATTCTCTCTGCC
GCCCACTGCTTCCAGGAGAGGTTTCCGCCCCACCACCTGACGGTGATCTTGGGCAGAACA
TACCGGGTGGTCCCTGGCGAGGAGGAGCAGAAATTTGAAGTCGAAAAATACATTGTCCAT
AAGGAATTCGATGATGACACTTACGACAATGACATTGCGCTGCTGCAGCTGAAATCGGAT
TCGTCCCGCTGTGCCCAGGAGAGCAGCGTGGTCCGCACTGTGTGCCTTCCCCCGGCGGAC
CTGCAGCTGCCGGACTGGACGGAGTGTGAGCTCTCCGGCTACGGCAAGCATGAGGCCTTG
TCTCCTTTCTATTCGGAGCGGCTGAAGGAGGCTCATGTCAGACTGTACCCATCCAGCCGC
TGCACATCACAACATTTACTTAACAGAACAGTCACCGACAACATGCTGTGTGCTGGAGAC
ACTCGGAGCGGCGGGCCCCAGGCAAACTTGCACGACGCCTGCCAGGGCGATTCGGGAGGC
CCCCTGGTGTGTCTGAACGATGGCCGCATGACTTTGGTGGGCATCATCAGCTGGGGCCTG
GGCTGTGGACAGAAGGATGTCCCGGGTGTGTACACAAAGGTTACCAACTACCTAGACTGG
ATTCGTGACAACATGCGACCG  1341
```

or a different DNA sequence within the scope of the degeneracy of the genetic code which codes for a corresponding t-PA derivative, is introduced into suitable host cells and the expression product is isolated from the culture medium or after lysis of the host cells.

2.  Process as claimed in claim 1,
    **wherein**
    the plasmid pA33 according to Fig. 1 is used.

3.  Process as claimed in claims 1 or 2,
    **wherein**
    prokaryotic cells, in particular E. coli are used as host cells.

4.  Process as claimed in claims 1, 2 or 3,
    **wherein**
    the yield of active protein is increased by isolating the "inclusion bodies" that form, solubilizing them by treatment with guanidine hydrochloride, followed by derivatization with oxidized glutathione and finally renaturation of the t-PA derivative by addition of L-arginine and GSH.

5.  Process as claimed in one of the claims 1 to 4,
    **wherein**
    one works in the presence of 25 to 1000 mmol/l L-arginine.

6. Process as claimed in one of the claims 1 to 5,
**wherein**
after the renaturation, the t-PA in the renaturation mixture is concentrated and subsequently a chromatographic purification by means of affinity chromatography on an ETI adsorber column is carried out.

7. Process as claimed in claim 6,
**wherein**
the chromatographic purification is carried out with the concentrate of the renaturation mixture which contains 25 to 1000 mmol/l, preferably 200 to 1000 mmol/l L-arginine.

8. Process as claimed in claim 7,
**wherein**
the concentrate used for the chromatographic purification is adjusted to a pH value of more than 7.0.

9. Process as claimed in one of the claims 6 to 8,
**wherein**
the elution is carried out at a pH value of 4 to 6.

10. Process for the construction of an expression plasmid which contains the DNA sequence

```
ATGTCTTACCAAGTGATCGATACCAGGGCCACGTGCTACGAGGACCAGGGCATCAGCTAC
AGGGGCACGTGGAGCACAGCGGAGAGTGGCGCCGAGTGCACCAACTGGAACAGCAGCGCG
TTGGCCCAGAAGCCCTACAGCGGGCGGAGGCCAGACGCCATCAGGCTGGGCCTGGGGAAC
CACAACTACTGCAGAAACCCAGATCGAGACTCAAAGCCCTGGTGCTACGTCTTTAAGGCG
GGGAAGTACAGCTCAGAGTTCTGCAGCACCCCTGCCTGCTCTGAGGGAAACAGTGACTGC
TACTTTGGGAATGGGTCAGCCTACCGTGGCACGCACAGCCTCACCGAGTCGGGTGCCTCC
TGCCTCCCGTGGAATTCCATGATCCTGATAGGCAAGGTTTACACAGCACAGAACCCCAGT
GCCCAGGCACTGGGCCTGGGCAAACATAATTACTGCCGGAATCCTGATGGGGATGCCAAG
CCCTGGTGCCACGTGCTGAAGAACCGCAGGCTGACGTGGGAGTACTGTGATGTGCCCTCC
TGCTCCACCTGCGGCCTGAGACAGTACAGCCAGCCTCAGTTTCGCATCAAAGGAGGGCTC
TTCGCCGACATCGCCTCCCACCCCTGGCAGGCTGCCATCTTTGCCAAGCACAGGAGGTCG
CCCGGAGAGCGGTTCCTGTGCGGGGGCATACTCATCAGCTCCTGCTGGATTCTCTCTGCC
GCCCACTGCTTCCAGGAGAGGTTTCCGCCCCACCACCTGACGGTGATCTTGGGCAGAACA
TACCGGGTGGTCCCTGGCGAGGAGGAGCAGAAATTTGAAGTCGAAAAATACATTGTCCAT
AAGGAATTCGATGATGACACTTACGACAATGACATTGCGCTGCTGCAGCTGAAATCGGAT
TCGTCCCGCTGTGCCCAGGAGAGCAGCGTGGTCCGCACTGTGTGCCTTCCCCCGGCGGAC
CTGCAGCTGCCGGACTGGACGGAGTGTGAGCTCTCCGGCTACGGCAAGCATGAGGCCTTG
TCTCCTTTCTATTCGGAGCGGCTGAAGGAGGCTCATGTCAGACTGTACCCATCCAGCCGC
TGCACATCACAACATTTACTTAACAGAACAGTCACCGACAACATGCTGTGTGCTGGAGAC
ACTCGGAGCGGCGGGCCCCAGGCAAACTTGCACGACGCCTGCCAGGGCGATTCGGGAGGC
CCCCTGGTGTGTCTGAACGATGGCCGCATGACTTTGGTGGGCATCATCAGCTGGGGCCTG
GGCTGTGGACAGAAGGATGTCCCGGGTGTGTACACAAAGGTTACCAACTACCTAGACTGG
ATTCGTGACAACATGCGACCG    1341
```

or a different DNA sequence within the scope of the degeneracy of the genetic code which codes for a

EP 0 400 545 B1

t-PA derivative which is not glycosylated and has the following amino acid sequence

(M)

Ser Tyr Gln Val Ile Asp Thr Arg Ala Thr Cys Tyr Glu Asp Gln Gly
                5                      10                      15

Ile Ser Tyr Arg Gly Thr Trp Ser Thr Ala Glu Ser Gly Ala Glu Cys
                20                      25                      30

Thr Asn Trp Asn Ser Ser Ala Leu Ala Gln Lys Pro Tyr Ser Gly Arg
            35                      40                      45

Arg Pro Asp Ala Ile Arg Leu Gly Leu Gly Asn His Asn Tyr Cys Arg
        50                      55                      60

Asn Pro Asp Arg Asp Ser Lys Pro Trp Cys Tyr Val Phe Lys Ala Gly
    65                      70                      75                      80

Lys Tyr Ser Ser Glu Phe Cys Ser Thr Pro Ala Cys Ser Glu Gly Asn
                85                      90                      95

Ser Asp Cys Tyr Phe Gly Asn Gly Ser Ala Tyr Arg Gly Thr His Ser
                100                     105                     110

Leu Thr Glu Ser Gly Ala Ser Cys Leu Pro Trp Asn Ser Met Ile Leu
            115                     120                     125

Ile Gly Lys Val Tyr Thr Ala Gln Asn Pro Ser Ala Gln Ala Leu Gly
        130                     135                     140

Leu Gly Lys His Asn Tyr Cys Arg Asn Pro Asp Gly Asp Ala Lys Pro
145                     150                     155                     160

Trp Cys His Val Leu Lys Asn Arg Arg Leu Thr Trp Glu Tyr Cys Asp
                165                     170                     175

Val Pro Ser Cys Ser Thr Cys Gly Leu Arg Gln Tyr Ser Gln Pro Gln
                180                     185                     190

Phe Arg Ile Lys Gly Gly Leu Phe Ala Asp Ile Ala Ser His Pro Trp
        195                     200                     205

Gln Ala Ala Ile Phe Ala Lys His Arg Arg Ser Pro Gly Glu Arg Phe
    210                     215                     220

Leu Cys Gly Gly Ile Leu Ile Ser Ser Cys Trp Ile Leu Ser Ala Ala
225                     230                     235                     240

31

```
His Cys Phe Gln Glu Arg Phe Pro Pro His His Leu Thr Val Ile Leu
             245                 250                       255

Gly Arg Thr Tyr Arg Val Val Pro Gly Glu Glu Glu Gln Lys Phe Glu
             260                 265                 270

Val Glu Lys Tyr Ile Val His Lys Glu Phe Asp Asp Asp Thr Tyr Asp
        275                 280                 285

Asn Asp Ile Ala Leu Leu Gln Leu Lys Ser Asp Ser Ser Arg Cys Ala
    290                 295                 300

Gln Glu Ser Ser Val Val Arg Thr Val Cys Leu Pro Pro Ala Asp Leu
305                 310                 315                 320

Gln Leu Pro Asp Trp Thr Glu Cys Glu Leu Ser Gly Tyr Gly Lys His
             325                 330                 335

Glu Ala Leu Ser Pro Phe Tyr Ser Glu Arg Leu Lys Glu Ala His Val
             340                 345                 350

Arg Leu Tyr Pro Ser Ser Arg Cys Thr Ser Gln His Leu Leu Asn Arg
        355                 360                 365

Thr Val Thr Asp Asn Met Leu Cys Ala Gly Asp Thr Arg Ser Gly Gly
    370                 375                 380

Pro Gln Ala Asn Leu His Asp Ala Cys Gln Gly Asp Ser Gly Gly Pro
385                 390                 395                 400

Leu Val Cys Leu Asn Asp Gly Arg Met Thr Leu Val Gly Ile Ile Ser
             405                 410                       415

Trp Gly Leu Gly Cys Gly Gln Lys Asp Val Pro Gly Val Tyr Thr Lys
             420                 425                 430

Val Thr Asn Tyr Leu Asp Trp Ile Arg Asp Asn Met Arg Pro
        435                 440                 445
```

or of plasmid pA33 according to Figure 1
**wherein,**
a DNA sequence which codes for the whole t-PA protein or for a derivative thereof that contains further regions of the t-PA protein in addition to the kringle I, kringle II and the protease domains is introduced into a plasmid and those domains which code for amino acids that are not present in the t-PA derivative are removed by site-directed mutagenesis.

**11.** Process as claimed in claim 10, wherein the corresponding cDNA is used as the DNA sequence for the t-PA protein or a derivative thereof.


**Revendications**
**Revendications pour les Etats contractants suivants : AT, BE, CH, LI, DE, DK, FR, GB, GR, IT, LU, NL, SE**

**1.** Dérivé de l'activateur du plasminogène tissulaire (t-PA), caractérisé en ce qu'il n'est pas glycosylé et présente la séquence d'acides aminés suivante:

(M)

```
Ser Tyr Gln Val Ile Asp Thr Arg Ala Thr Cys Tyr Glu Asp Gln
                  5                    10                   15
Gly Ile Ser Tyr Arg Gly Thr Trp Ser Thr Ala Glu Ser Gly Ala
                      20                   25                  30
Glu Cys Thr Asn Trp Asn Ser Ser Ala Leu Ala Gln Lys Pro Tyr
                      35                   40                  45
Ser Gly Arg Arg Pro Asp Ala Ile Arg Leu Gly Leu Gly Asn His
                      50                   55                  60
Asn Tyr Cys Arg Asn Pro Asp Arg Asp Ser Lys Pro Trp Cys Tyr
                      65                   70                  75
Val Phe Lys Ala Gly Lys Tyr Ser Ser Glu Phe Cys Ser Thr Pro
                      80                   85                  90
Ala Cys Ser Glu Gly Asn Ser Asp Cys Tyr Phe Gly Asn Gly Ser
                      95                  100                 105
Ala Tyr Arg Gly Thr His Ser Leu Thr Glu Ser Gly Ala Ser Cys
                     110                  115                 120
Leu Pro Trp Asn Ser Met Ile Leu Ile Gly Lys Val Tyr Thr Ala
                     125                  130                 135
Gln Asn Pro Ser Ala Gln Ala Leu Gly Leu Gly Lys His Asn Tyr
                     140                  145                 150
Cys Arg Asn Pro Asp Gly Asp Ala Lys Pro Trp Cys His Val Leu
                     155                  160                 165
Lys Asn Arg Arg Leu Thr Trp Glu Tyr Cys Asp Val Pro Ser Cys
                     170                  175                 180
Ser Thr Cys Gly Leu Arg Gln Tyr Ser Gln Pro Gln Phe Arg Ile
                     185                  190                 195
```

```
Lys Gly Gly Leu Phe Ala Asp Ile Ala Ser His Pro Trp Gln Ala
              200                 205                 210

Ala Ile Phe Ala Lys His Arg Arg Ser Pro Gly Glu Arg Phe Leu
              215                 220                 225

Cys Gly Gly Ile Leu Ile Ser Ser Cys Trp Ile Leu Ser Ala Ala
              230                 235                 240

His Cys Phe Gln Glu Arg Phe Pro Pro His His Leu Thr Val Ile
              245                 250                 255

Leu Gly Arg Thr Tyr Arg Val Val Pro Gly Glu Glu Glu Gln Lys
              260                 265                 270

Phe Glu Val Glu Lys Tyr Ile Val His Lys Glu Phe Asp Asp Asp
              275                 280                 285

Thr Tyr Asp Asn Asp Ile Ala Leu Leu Gln Leu Lys Ser Asp Ser
              290                 295                 300

Ser Arg Cys Ala Gln Glu Ser Ser Val Val Arg Thr Val Cys Leu
              305                 310                 315

Pro Pro Ala Asp Leu Gln Leu Pro Asp Trp Thr Glu Cys Glu Leu
              320                 325                 330

Ser Gly Tyr Gly Lys His Glu Ala Leu Ser Pro Phe Tyr Ser Glu
              335                 340                 345

Arg Leu Lys Glu Ala His Val Arg Leu Tyr Pro Ser Ser Arg Cys
              350                 355                 360

Thr Ser Gln His Leu Leu Asn Arg Thr Val Thr Asp Asn Met Leu
              365                 370                 375

Cys Ala Gly Asp Thr Arg Ser Gly Gly Pro Gln Ala Asn Leu His
              380                 385                 390

Asp Ala Cys Gln Gly Asp Ser Gly Gly Pro Leu Val Cys Leu Asn
              395                 400                 405

Asp Gly Arg Met Thr Leu Val Gly Ile Ile Ser Trp Gly Leu Gly
              410                 415                 420

Cys Gly Gln Lys Asp Val Pro Gly Val Tyr Thr Lys Val Thr Asn
              425                 430                 435

Tyr Leu Asp Trp Ile Arg Asp Asn Met Arg Pro
              440                 445
```

2.  Séquence d'ADN caractérisée en ce qu'elle code un dérivé du t-PA selon la revendication 1 et contient la séquence suivante:

34

```
ATGTCTTACCAAGTGATCGATACCAGGGCCACGTGCTACGAGGACCAGGGCATCAGCTAC
AGGGGCACGTGGAGCACAGCGGAGAGTGGCGCCGAGTGCACCAACTGGAACAGCAGCGCG
TTGGCCCAGAAGCCCTACAGCGGGCGGAGGCCAGACGCCATCAGGCTGGGCCTGGGGAAC
CACAACTACTGCAGAAACCCAGATCGAGACTCAAAGCCCTGGTGCTACGTCTTTAAGGCG
GGGAAGTACAGCTCAGAGTTCTGCAGCACCCCTGCCTGCTCTGAGGGAAACAGTGACTGC
TACTTTGGGAATGGGTCAGCCTACCGTGGCACGCACAGCCTCACCGAGTCGGGTGCCTCC
TGCCTCCCGTGGAATTCCATGATCCTGATAGGCAAGGTTTACACAGCACAGAACCCCAGT
GCCCAGGCACTGGGCCTGGGCAAACATAATTACTGCCGGAATCCTGATGGGGATGCCAAG
CCCTGGTGCCACGTGCTGAAGAACCGCAGGCTGACGTGGGAGTACTGTGATGTGCCCTCC
TGCTCCACCTGCGGCCTGAGACAGTACAGCCAGCCTCAGTTTCGCATCAAAGGAGGGCTC
TTCGCCGACATCGCCTCCCACCCCTGGCAGGCTGCCATCTTTGCCAAGCACAGGAGGTCG
CCCGGAGAGCGGTTCCTGTGCGGGGGCATACTCATCAGCTCCTGCTGGATTCTCTCTGCC
GCCCACTGCTTCCAGGAGAGGTTTCCGCCCCACCACCTGACGGTGATCTTGGGCAGAACA
TACCGGGTGGTCCCTGGCGAGGAGGAGCAGAAATTTGAAGTCGAAAAATACATTGTCCAT
AAGGAATTCGATGATGACACTTACGACAATGACATTGCGCTGCTGCAGCTGAAATCGGAT
TCGTCCCGCTGTGCCCAGGAGAGCAGCGTGGTCCGCACTGTGTGCCTTCCCCCGGCGGAC
CTGCAGCTGCCGGACTGGACGGAGTGTGAGCTCTCCGGCTACGGCAAGCATGAGGCCTTG
TCTCCTTTCTATTCGGAGCGGCTGAAGGAGGCTCATGTCAGACTGTACCCATCCAGCCGC
TGCACATCACAACATTTACTTAACAGAACAGTCACCGACAACATGCTGTGTGCTGGAGAC
ACTCGGAGCGGCGGGCCCCAGGCAAACTTGCACGACGCCTGCCAGGGCGATTCGGGAGGC
CCCCTGGTGTGTCTGAACGATGGCCGCATGACTTTGGTGGGCATCATCAGCTGGGGCCTG
GGCTGTGGACAGAAGGATGTCCCGGGTGTGTACACAAAGGTTACCAACTACCTAGACTGG
ATTCGTGACAACATGCGACCG    1341
```

**3.** Plasmide d'expression caractérisé en ce qu'il contient la séquence d'ADN selon la revendication 2, ou une séquence d'ADN différente de celle-ci dans le cadre de la dégénérescence du code génétique, qui code une protéine selon la revendication 1.

**4.** Plasmide pA33 selon la figure 1.

**5.** Procédé de production d'un plasmide d'expression selon l'une des revendications 3 ou 4, caractérisé en ce que l'on introduit dans un plasmide une séquence d'ADN qui code la protéine t-PA totale ou un dérivé de celle-ci qui, outre les domaines kringel I, kringel II et de protéase, présente encore d'autres domaines de la protéine t-PA et on élimine par mutagenèse dirigée les domaines qui codent des acides aminés qui ne sont pas présents dans le dérivé du t-PA selon la revendication 1.

**6.** Procédé selon la revendication 5, caractérisé en ce que l'on utilise l'ADNc correspondant code séquence d'ADN pour la protéine t-PA ou un dérivé de celle-ci.

**7.** Procédé de production d'un dérivé du t-PA selon la revendication 1, caractérisé en ce que l'on introduit dans des cellules hôtes appropriées un plasmide selon l'une des revendications 3 ou 4 et on obtient le produit d'expression à partir du milieu de culture ou après lyse des cellules hôtes.

**8.** Procédé selon la revendication 7, caractérisé en ce que l'on utilise comme cellules hôtes des cellules procaryotiques, en particulier E. coli.

**9.** Procédé selon la revendication 8, caractérisé en ce que, pour augmenter le rendement en protéine active, on sépare les "inclusion bodies" formés, on les solubilise par traitement avec le chlorhydrate de guanidine, puis on les transforme en dérivé avec du glutathion oxydé et enfin on renature le dérivé du t-PA par addition de L-arginine et de GSH.

**10.** Procédé selon l'une des revendications 7 à 9, caractérisé en ce que l'on opère en présence de 25 à 1000 mmol/l de L-arginine.

**11.** Procédé selon l'une des revendications 7 à 10, caractérisé en ce que, après la renaturation, on concentre le dérivé du t-PA dans la charge de renaturation puis on réalise une purification chromatographique par chromatographie d'affinité sur une colonne de ETI-adsorbant.

**12.** Procédé selon la revendication 11, caractérisé en ce que l'on réalise la purification chromatographique avec le concentré de la charge de renaturation qui contient 25 à 1000 mmol/l, de préférence 200 à 1000 mmol/l, de L-arginine.

**13.** Procédé selon la revendication 12, caractérisé en ce que l'on ajuste à un pH supérieur à 7,0 le concentré utilisé pour la purification chromatographique.

**14.** Procédé selon l'une des revendications 11 à 13, caractérisé en ce que l'on réalise l'élution à un pH de 4 à 6.

**15.** Agent pour dissoudre les caillots sanguins, contenant un dérivé du t-PA selon la revendication 1.

**Revendications pour l'Etat contractant suivant : ES**

**1.** Procédé de production d'un dérivé du t-PA qui n'est pas glycosylé et présente la séquence d'acides aminés suivante:

(M)

```
Ser Tyr Gln Val Ile Asp Thr Arg Ala Thr Cys Tyr Glu Asp Gln
                 5                   10                  15
Gly Ile Ser Tyr Arg Gly Thr Trp Ser Thr Ala Glu Ser Gly Ala
                20                   25                  30
Glu Cys Thr Asn Trp Asn Ser Ser Ala Leu Ala Gln Lys Pro Tyr
                35                   40                  45
Ser Gly Arg Arg Pro Asp Ala Ile Arg Leu Gly Leu Gly Asn His
                50                   55                  60
Asn Tyr Cys Arg Asn Pro Asp Arg Asp Ser Lys Pro Trp Cys Tyr
                65                   70                  75
Val Phe Lys Ala Gly Lys Tyr Ser Ser Glu Phe Cys Ser Thr Pro
                80                   85                  90
Ala Cys Ser Glu Gly Asn Ser Asp Cys Tyr Phe Gly Asn Gly Ser
                95                  100                 105
Ala Tyr Arg Gly Thr His Ser Leu Thr Glu Ser Gly Ala Ser Cys
                110                 115                 120
Leu Pro Trp Asn Ser Met Ile Leu Ile Gly Lys Val Tyr Thr Ala
                125                 130                 135
Gln Asn Pro Ser Ala Gln Ala Leu Gly Leu Gly Lys His Asn Tyr
                140                 145                 150
Cys Arg Asn Pro Asp Gly Asp Ala Lys Pro Trp Cys His Val Leu
                155                 160                 165
Lys Asn Arg Arg Leu Thr Trp Glu Tyr Cys Asp Val Pro Ser Cys
                170                 175                 180
Ser Thr Cys Gly Leu Arg Gln Tyr Ser Gln Pro Gln Phe Arg Ile
                185                 190                 195
```

37

```
Lys Gly Gly Leu Phe Ala Asp Ile Ala Ser His Pro Trp Gln Ala
                200                 205                 210
Ala Ile Phe Ala Lys His Arg Arg Ser Pro Gly Glu Arg Phe Leu
                215                 220                 225
Cys Gly Gly Ile Leu Ile Ser Ser Cys Trp Ile Leu Ser Ala Ala
                230                 235                 240
His Cys Phe Gln Glu Arg Phe Pro Pro His His Leu Thr Val Ile
                245                 250                 255
Leu Gly Arg Thr Tyr Arg Val Val Pro Gly Glu Glu Glu Gln Lys
                260                 265                 270
Phe Glu Val Glu Lys Tyr Ile Val His Lys Glu Phe Asp Asp Asp
                275                 280                 285
Thr Tyr Asp Asn Asp Ile Ala Leu Leu Gln Leu Lys Ser Asp Ser
                290                 295                 300
Ser Arg Cys Ala Gln Glu Ser Ser Val Val Arg Thr Val Cys Leu
                305                 310                 315
Pro Pro Ala Asp Leu Gln Leu Pro Asp Trp Thr Glu Cys Glu Leu
                320                 325                 330
Ser Gly Tyr Gly Lys His Glu Ala Leu Ser Pro Phe Tyr Ser Glu
                335                 340                 345
Arg Leu Lys Glu Ala His Val Arg Leu Tyr Pro Ser Ser Arg Cys
                350                 355                 360
Thr Ser Gln His Leu Leu Asn Arg Thr Val Thr Asp Asn Met Leu
                365                 370                 375
Cys Ala Gly Asp Thr Arg Ser Gly Gly Pro Gln Ala Asn Leu His
                380                 385                 390
Asp Ala Cys Gln Gly Asp Ser Gly Gly Pro Leu Val Cys Leu Asn
                395                 400                 405
Asp Gly Arg Met Thr Leu Val Gly Ile Ile Ser Trp Gly Leu Gly
                410                 415                 420
Cys Gly Gln Lys Asp Val Pro Gly Val Tyr Thr Lys Val Thr Asn
                425                 430                 435
Tyr Leu Asp Trp Ile Arg Asp Asn Met Arg Pro
                440                 445
```

caractérisé en ce que l'on introduit dans des cellules hôtes appropriées un plasmide d'expression qui contient la séquence d'ADN:

```
ATGTCTTACCAAGTGATCGATACCAGGGCCACGTGCTACGAGGACCAGGGCATCAGCTAC
AGGGGCACGTGGAGCACAGCGGAGAGTGGCGCCGAGTGCACCAACTGGAACAGCAGCGCG
TTGGCCCAGAAGCCCTACAGCGGGCGGAGGCCAGACGCCATCAGGCTGGGCCTGGGGAAC
CACAACTACTGCAGAAACCCAGATCGAGACTCAAAGCCCTGGTGCTACGTCTTTAAGGCG
GGGAAGTACAGCTCAGAGTTCTGCAGCACCCCTGCCTGCTCTGAGGGAAACAGTGACTGC
TACTTTGGGAATGGGTCAGCCTACCGTGGCACGCACAGCCTCACCGAGTCGGGTGCCTCC
TGCCTCCCGTGGAATTCCATGATCCTGATAGGCAAGGTTTACACAGCACAGAACCCCAGT
GCCCAGGCACTGGGCCTGGGCAAACATAATTACTGCCGGAATCCTGATGGGGATGCCAAG
CCCTGGTGCCACGTGCTGAAGAACCGCAGGCTGACGTGGGAGTACTGTGATGTGCCCTCC
TGCTCCACCTGCGGCCTGAGACAGTACAGCCAGCCTCAGTTTCGCATCAAAGGAGGGCTC
TTCGCCGACATCGCCTCCCACCCCTGGCAGGCTGCCATCTTTGCCAAGCACAGGAGGTCG
CCCGGAGAGCGGTTCCTGTGCGGGGGCATACTCATCAGCTCCTGCTGGATTCTCTCTGCC
GCCCACTGCTTCCAGGAGAGGTTTCCGCCCCACCACCTGACGGTGATCTTGGGCAGAACA
TACCGGGTGGTCCCTGGCGAGGAGGAGCAGAAATTTGAAGTCGAAAAATACATTGTCCAT
AAGGAATTCGATGATGACACTTACGACAATGACATTGCGCTGCTGCAGCTGAAATCGGAT
TCGTCCCGCTGTGCCCAGGAGAGCAGCGTGGTCCGCACTGTGTGCCTTCCCCGGCGGAC
CTGCAGCTGCCGGACTGGACGGAGTGTGAGCTCTCCGGCTACGGCAAGCATGAGGCCTTG
TCTCCTTTCTATTCGGAGCGGCTGAAGGAGGCTCATGTCAGACTGTACCCATCCAGCCGC
TGCACATCACAACATTTACTTAACAGAACAGTCACCGACAACATGCTGTGTGCTGGAGAC
ACTCGGAGCGGCGGGCCCCAGGCAAACTTGCACGACGCCTGCCAGGGCGATTCGGGAGGC
CCCCTGGTGTGTCTGAACGATGGCCGCATGACTTTGGTGGGCATCATCAGCTGGGGCCTG
GGCTGTGGACAGAAGGATGTCCCGGGTGTGTACACAAAGGTTACCAACTACCTAGACTGG
ATTCGTGACAACATGCGACCG    1341
```

ou une séquence d'ADN différente de celle-ci dans le cadre de la dégénérescence du code génétique, qui code un dérivé du t-PA correspondant et on obtient le produit d'expression à partir du milieu de culture ou après lyse des cellules hôtes.

2.  Procédé selon la revendication 1, caractérisé en ce que l'on utilise le plasmide pA33 selon la figure 1.

3.  Procédé selon la revendication 1 ou 2, caractérisé en ce que l'on utilise comme cellules hôtes des cellules procaryotiques, en particulier E. coli.

4.  Procédé selon la revendication 1, 2 ou 3, caractérisé en ce que, pour augmenter le rendement en protéine active, on sépare les "inclusion bodies" formés, on les solubilise par traitement avec le chlorhydrate de guanidine, puis on les transforme en dérivé avec du glutathion oxydé et enfin on renature le dérivé du t-PA par addition de L-arginine et de GSH.

5.  Procédé selon l'une des revendications 1 à 4, caractérisé en ce que l'on opère en présence de 25 à 1000 mmol/l de L-arginine.

6.  Procédé selon l'une des revendications 1 à 5, caractérisé en ce que, après la renaturation, on concentre le dérivé du t-PA dans la charge de renaturation puis on réalise une purification chromatographique par chromatographie d'affinité sur une colonne de ETI-adsorbant.

7.  Procédé selon la revendication 6, caractérisé en ce que l'on réalise la purification chromatographique avec le concentré de la charge de renaturation qui contient 25 à 1000 mmol/l, de préférence 200 à 1000 mmol/l, de L-arginine.

8. Procédé selon la revendication 7, caractérisé en ce que l'on ajuste à un pH supérieur à 7,0 le concentré utilisé pour la purification chromatographique.

9. Procédé selon l'une des revendications 6 à 8, caractérisé en ce que l'on réalise l'élution à un pH de 4 à 6.

10. Procédé de production d'un plasmide d'expression qui contient la séquence d'ADN:

```
ATGTCTTACCAAGTGATCGATACCAGGGCCACGTGCTACGAGGACCAGGGCATCAGCTAC
AGGGGCACGTGGAGCACAGCGGAGAGTGGCGCCGAGTGCACCAACTGGAACAGCAGCGCG
TTGGCCCAGAAGCCCTACAGCGGGCGGAGGCCAGACGCCATCAGGCTGGGCCTGGGGAAC
CACAACTACTGCAGAAACCCAGATCGAGACTCAAAGCCCTGGTGCTACGTCTTTAAGGCG
GGGAAGTACAGCTCAGAGTTCTGCAGCACCCCTGCCTGCTCTGAGGGAAACAGTGACTGC
TACTTTGGGAATGGGTCAGCCTACCGTGGCACGCACAGCCTCACCGAGTCGGGTGCCTCC
TGCCTCCCGTGGAATTCCATGATCCTGATAGGCAAGGTTTACACAGCACAGAACCCCAGT
GCCCAGGCACTGGGCCTGGGCAAACATAATTACTGCCGGAATCCTGATGGGGATGCCAAG
CCCTGGTGCCACGTGCTGAAGAACCGCAGGCTGACGTGGGAGTACTGTGATGTGCCCTCC
TGCTCCACCTGCGGCCTGAGACAGTACAGCCAGCCTCAGTTTCGCATCAAAGGAGGGCTC
TTCGCCGACATCGCCTCCCACCCCTGGCAGGCTGCCATCTTTGCCAAGCACAGGAGGTCG
CCCGGAGAGCGGTTCCTGTGCGGGGGCATACTCATCAGCTCCTGCTGGATTCTCTCTGCC
GCCCACTGCTTCCAGGAGAGGTTTCCGCCCCACCACCTGACGGTGATCTTGGGCAGAACA
TACCGGGTGGTCCCTGGCGAGGAGGAGCAGAAATTTGAAGTCGAAAAATACATTGTCCAT
AAGGAATTCGATGATGACACTTACGACAATGACATTGCGCTGCTGCAGCTGAAATCGGAT
TCGTCCCGCTGTGCCCAGGAGAGCAGCGTGGTCCGCACTGTGTGCCTTCCCCCGGCGGAC
CTGCAGCTGCCGGACTGGACGGAGTGTGAGCTCTCCGGCTACGGCAAGCATGAGGCCTTG
TCTCCTTTCTATTCGGAGCGGCTGAAGGAGGCTCATGTCAGACTGTACCCATCCAGCCGC
TGCACATCACAACATTTACTTAACAGAACAGTCACCGACAACATGCTGTGTGCTGGAGAC
ACTCGGAGCGGCGGGCCCCAGGCAAACTTGCACGACGCCTGCCAGGGCGATTCGGGAGGC
CCCCTGGTGTGTCTGAACGATGGCCGCATGACTTTGGTGGGCATCATCAGCTGGGGCCTG
GGCTGTGGACAGAAGGATGTCCCGGGTGTGTACACAAAGGTTACCAACTACCTAGACTGG
ATTCGTGACAACATGCGACCG    1341
```

ou une séquence d'ADN différente de celle-ci dans le cadre de la dégénérescence du code génétique, qui code une protéine qui n'est pas glycosylée et présente la séquence d'acides aminés suivante:

(M)

```
Ser Tyr Gln Val Ile Asp Thr Arg Ala Thr Cys Tyr Glu Asp Gln
                      5                  10                  15
Gly Ile Ser Tyr Arg Gly Thr Trp Ser Thr Ala Glu Ser Gly Ala
                     20                  25                  30
Glu Cys Thr Asn Trp Asn Ser Ser Ala Leu Ala Gln Lys Pro Tyr
                     35                  40                  45
```

```
Ser Gly Arg Arg Pro Asp Ala Ile Arg Leu Gly Leu Gly Asn His
                50                  55                  60
Asn Tyr Cys Arg Asn Pro Asp Arg Asp Ser Lys Pro Trp Cys Tyr
                65                  70                  75
Val Phe Lys Ala Gly Lys Tyr Ser Ser Glu Phe Cys Ser Thr Pro
                80                  85                  90
Ala Cys Ser Glu Gly Asn Ser Asp Cys Tyr Phe Gly Asn Gly Ser
                95                 100                 105
Ala Tyr Arg Gly Thr His Ser Leu Thr Glu Ser Gly Ala Ser Cys
               110                 115                 120
Leu Pro Trp Asn Ser Met Ile Leu Ile Gly Lys Val Tyr Thr Ala
               125                 130                 135
Gln Asn Pro Ser Ala Gln Ala Leu Gly Leu Gly Lys His Asn Tyr
               140                 145                 150
Cys Arg Asn Pro Asp Gly Asp Ala Lys Pro Trp Cys His Val Leu
               155                 160                 165
Lys Asn Arg Arg Leu Thr Trp Glu Tyr Cys Asp Val Pro Ser Cys
               170                 175                 180
Ser Thr Cys Gly Leu Arg Gln Tyr Ser Gln Pro Gln Phe Arg Ile
               185                 190                 195
Lys Gly Gly Leu Phe Ala Asp Ile Ala Ser His Pro Trp Gln Ala
               200                 205                 210
Ala Ile Phe Ala Lys His Arg Arg Ser Pro Gly Glu Arg Phe Leu
               215                 220                 225
Cys Gly Gly Ile Leu Ile Ser Ser Cys Trp Ile Leu Ser Ala Ala
               230                 235                 240
His Cys Phe Gln Glu Arg Phe Pro Pro His His Leu Thr Val Ile
               245                 250                 255
Leu Gly Arg Thr Tyr Arg Val Val Pro Gly Glu Glu Glu Gln Lys
               260                 265                 270
Phe Glu Val Glu Lys Tyr Ile Val His Lys Glu Phe Asp Asp Asp
               275                 280                 285
Thr Tyr Asp Asn Asp Ile Ala Leu Leu Gln Leu Lys Ser Asp Ser
               290                 295                 300
```

```
Ser Arg Cys Ala Gln Glu Ser Ser Val Val Arg Thr Val Cys Leu
                305                     310                     315
Pro Pro Ala Asp Leu Gln Leu Pro Asp Trp Thr Glu Cys Glu Leu
                320                     325                     330
Ser Gly Tyr Gly Lys His Glu Ala Leu Ser Pro Phe Tyr Ser Glu
                335                     340                     345
Arg Leu Lys Glu Ala His Val Arg Leu Tyr Pro Ser Ser Arg Cys
                350                     355                     360
Thr Ser Gln His Leu Leu Asn Arg Thr Val Thr Asp Asn Met Leu
                365                     370                     375
Cys Ala Gly Asp Thr Arg Ser Gly Gly Pro Gln Ala Asn Leu His
                380                     385                     390
Asp Ala Cys Gln Gly Asp Ser Gly Gly Pro Leu Val Cys Leu Asn
                395                     400                     405
Asp Gly Arg Met Thr Leu Val Gly Ile Ile Ser Trp Gly Leu Gly
                410                     415                     420
Cys Gly Gln Lys Asp Val Pro Gly Val Tyr Thr Lys Val Thr Asn
                425                     430                     435
Tyr Leu Asp Trp Ile Arg Asp Asn Met Arg Pro
                440                     445
```

ou du plasmide pA33 selon la figure 1, caractérisé en ce que l'on introduit dans un plasmide une séquence d'ADN qui code la protéine t-PA totale ou un dérivé de celle-ci qui, outre les domaines kringel I, kringel II et de protéase, présente encore d'autres domaines de la protéine t-PA et on élimine par mutagenèse dirigée les domaines qui codent des acides aminés qui ne sont pas présents dans le dérivé du t-PA.

**11.** Procédé selon la revendication 10, caractérisé en ce que l'on utilise l'ADNc correspondant comme séquence d'ADN pour la protéine t-PA ou un dérivé de celle-ci.

# Fig.1

pePa 133
(t-PA)

pACYC 177

TM

Ap$^R$

EcoRI

EcoRI

EcoRI

P$_{tac}$

Km$^R$

XhoI

Deletion der
Finger-und der
EGF-Domäne

Mutagenese – Primer
5'TAC CAA GTG ATC GAT ACC AGG GCC 3'

pA 33
(K1 K2 P)

pACYC 177

TM

Ap$^R$

EcoRI

EcoRI

EcoRI

P$_{tac}$

Km$^R$

XhoI

Finger-Domäne (F)
EGF-Domane (E)
Kringel I-Domäne (K1)
Kringel II-Domäne (K2)
Protease-Domäne (P)

P$_{tac}$ = tac-Promoter
TM = Terminator
Ap$^R$ = Ampicillin-Resistenz
Km$^R$ = Kanamycin-Resistenz

FIG.2

Zeitlicher Verlauf der t-PA-Aktivitäts-Plasmakonzentration (lin.) nach einer i.v. Bolusinjektion (über 1 min) von 200 000 IU/kg KG von Actilyse[R] oder K1K2P/Pro in narkotisierten Kaninchen

⊡ = ACTILYSE ®     200 000 IU/kg
    N = 6

□ = MUTEIN K1K2P/PRO     200 000 IU/kg
    N = 6

arithmetische Mittelwerte

t-PA-Aktivität

IU/ml

Zeit

Stunden

EP 0 400 545 B1

# FIG. 3

Zeitlicher Verlauf der t-PA-Aktivitäts-Plasmakonzentration (log.) nach einer i.v. Bolusinjektion (über 1min) von 200 000 IU/kg KG von Actilyse[R] oder R1R2P/Pro in narkotisierten Kaninchen

□ = ACTILYSE ®        200 000 IU/kg
    N = 6

■ = MUTEIN K1K2P/PRO   200 000 IU/kg
    N = 6

arithmetische Mittelwerte

t-PA-Aktivität

IU/ml

Zeit

Stunden

EP 0 400 545 B1